# EUROPEAN PATENT APPLICATION

(11) **EP 4 803 917 A2**
(43) Date of publication of application: **09.09.2026**
(21) Application number: 26181129.3
(22) Date of filing: 13.02.2023
(51) Int. Cl.: G01R 33/02

(54) **MAGNETIC COUPLING AND DETECTION MECHANISM FOR A PUMP DEVICE**

(30) Priority: 04.03.2022 US 202263316641 P
(62) Document number(s) of the earlier application(s) in accordance with Art. 76 EPC: 23763082.7 / 4 486 412
(71) Applicant: Neuroderm, Ltd., 7670212 Rehovot (IL)
(72) Inventor: FINKELSTEIN, Aharon, 9908903 Bet Shemesh (IL); CHEN, Lior Bar, 7681801 Ganei Tal (IL); MANE, Nitsan, 4940740 Petah-Tikva (IL)
(74) Representative: HGF

(57) **Abstract**

A bi-functional magnetic mechanism generates a magnetic attraction force to magnetically engage a disposable and reusable parts of a pump device, and, at the same time, the bi- functional magnetic mechanism uses the same magnetic field to detect an engagement between the two parts of the pump device. The mechanism includes a permanent magnet to produce magnetic field(s) that zero out a net magnetic field at a sensor, an asymmetrical metal plate (AMP) to magnetically disrupt (deflect, redirect) the magnetic field(s) at the sensor, and a magnetic field sensor to sense the magnetic disruption. The magnet and the sensor are incorporated in the reusable part of the pump device such that the magnetic field sensor is circumferentially surrounded by the magnet. The AMP is incorporated in the disposable part such that the AMP subtends the sensor (i.e., adjacent to the sensor) when the disposable part and reusable part are engaged.

## Description

### FIELD OF THE INVENTION

The present invention generally relates to systems and methods for coupling a disposable part ("DP") (e.g., liquid drug container(s)/reservoir(s)) of a drug delivery device (pump device) to a reusable part ("RP") of the pump device. More specifically, the present invention relates to magnetic coupling mechanisms for magnetically releasably coupling (releasably attaching/engaging) a DP to a RP of a drug delivery device, and to magnetic-field based detection methods for detecting engagement (coupling/attachment) and disengagement (decoupling), and optionally engagement orientation, between the DP and the RP of the drug delivery device.

### BACKGROUND

Some liquid drug delivery systems are two-part systems including a RP, which typically includes, among other things, an electric motor and a gear system that is driven by the electric motor, and a DP that typically includes liquid drug reservoir(s) and a gear-driven plunger rod to expel drug out of the reservoir(s). NeuroDerm Ltd. (a company based in Israel), for example, has developed a proprietary small two-part wearable infusion drug delivery device to deliver liquid drug to Parkinson disease (PD) patients subcutaneously.

Ability to detect, by a controller of the pump device, in conjunction with a sensor system, when the DP and the RP are properly engaged has benefits, for example in terms of safety of operation of the liquid drug delivery system, ensuring accurate drug dosing and avoiding various mechanical issues that may result from mechanical wear (that may change mechanical tolerances,) or damage. For example, engaging a DP of a pump device with a RP of the pump device should be a prerequisite to safe operation of the pump device.

Some pump devices use a magnetic field source and a magnetic field sensor to sense when the DP and the RP of the pump device are properly engaged. Typically to these devices, the DP includes a magnet as a magnetic field source, and the RP includes a magnetic field sensor. Using this kind of 'magnet-sensor' configuration, the magnetic field that is sensed by the magnetic field sensor is maximal when the DP and the RP are engaged, and, conversely, the magnetic field that is sensed by the magnetic field sensor is minimal when the DP and the RP are pulled away from one another. So, a decision regarding the state of engagement of the DP and the RP is made (e.g., by a controller) accordingly. Incorporating a magnet into the DP is wasteful because each DP requires a magnet, and, in addition, all magnets would have to be magnetized in exactly the same way in order to ensure that all pump devices perform in the same way.

Typically, a DP of a pump device that includes a magnet and a RP of the pump device that includes a magnetic field sensor are attached by using a mechanical coupling device or connector, for example a snap-fit mechanism (e.g., cantilever snap-fit), mating threaded elements or a bayonet connector. It would be beneficial to have a pump device where a magnet may be used for simultaneously releasably coupling the DP to the RP of the pump device and sensing when the DP and RP of the pump device are engaged and disengaged. In addition, it would be beneficial to have a pump device that improves usability (ease of use) for patients suffering from motor impairment, in particular patients who are unable to operate a mechanism that requires movement precision and accuracy, or physical strength, or both movement precision/accuracy and physical strength, as is often the case with PD patients.

### SUMMARY OF THE INVENTION

A bifunctional magnetic mechanism generates a magnetic attraction force to magnetically engage a disposable and reusable parts of a pump device, and, at the same time, the bifunctional magnetic mechanism uses the same magnetic field to detect an engagement state between the two parts of the pump device. The bifunctional magnetic mechanism includes a permanent magnet to produce magnetic field(s) that zero out a net magnetic field at a sensor, an asymmetrical metal plate (AMP) to magnetically disrupt (deflect, redirect) the magnetic field(s) at the sensor, and a magnetic field sensor to sense the magnetic disruption/deflection. The magnet and the sensor are incorporated in the RP of the pump device such that the magnetic field sensor is circumferentially surrounded by the magnet. The AMP is incorporated in the DP such that the AMP subtends the sensor (i.e., is adjacent to the sensor in functionally optimized manner) when the DP and RP are engaged. (The design (geometry and relative position) of the two parts (AMP and magnet/sensor, including the other parts supporting them) ensures that the AMP is centered in front of the magnet/sensor in an optimal manner to ensure optimal operation of the mechanism that is disclosed herein.

The pump device for delivering fluid medicament(s) to a user includes a RP and a DP for engagement with the RP. The RP includes, among other things, a magnetic field sensor having a magnetic field sensing area, a magnet that is circumferentially surrounding the magnetic field sensor and magnetized to produce one or more magnetic fields in direction(s) that zero out a net magnetic field that is sensed by the magnetic field sensor, and a controller to read an output value of the magnetic field sensor. The DP is magnetically engageable with the RP and includes a metal plate that is magnetically attractable to the magnet to magnetically engage the DP with the RP, and, during the engagement, to magnetically deflect one or more of the one or more magnetic fields at the magnetic field sensing area such that the net magnetic field sensed by the magnetic field sensor is greater than zero. The controller is configured to determine an engagement state between the DP and the RP from an output value (***Sa***) of the magnetic field sensor that corresponds to the sensed net magnetic field.

Each of the magnetic field sensing area, the magnet and the metal plate form a plane that coincides with, or is parallel to, an X-Z plane of the Cartesian coordinate system and perpendicular to the Y-axis of the Cartesian coordinate system. The magnet includes a central aperture, and the magnetic field sensor is centered in the aperture of the magnet at a point that coincides with the origin of the Cartesian coordinate system. The metal plate is asymmetrical with respect to an asymmetry line coinciding with the Z-axis, and the asymmetry line divides the metal plate into a primary section and an auxiliary section.

The primary section of the asymmetrical metal plate (AMP) is configured to induce a magnetic attraction force between the primary section and the magnet when the DP is brought into proximity to the RP, and, at the same time, to deflect the one or more of the one or more magnetic fields at or near the sensor's sensing area. The auxiliary section of the AMP is configured to induce a magnetic attraction force between the auxiliary section and the magnet when the DP is brought into proximity to the RP. The metal plate unevenly deflects the one or more of the one or more magnetic fields to make the deflection detectable by the controller. The metal plate may be configured such that the magnetic field sensor does not enter saturation when the DP is engaged with the RP in order to enable the controller to distinguish between the engagement state of the DP and RP, and a faulty condition that results in the magnetic field sensor entering saturation.

The DP may include one medicament reservoir, and the controller may compare the output value (***Sa***) of the magnetic field sensor to a threshold value ***(Sth)*** to determine the engagement state of the DP and RP. The controller may check the value of ***Sa*** once every ***t1*** seconds when the disposable and reusable parts of the pump device are engaged or the pump device actually delivers medicament to a patient, and once every ***t2*** seconds (where ***t2>t1*)** when the disposable and reusable parts of the pump device are disengaged or the pump device does not deliver medicament to the patient. The value of ***t1*** may be, for example, 0.01 second, 0.5 second, 1.0 second, etc., and the value of ***t2*** may be, for example, 3.0 seconds, 5.0 seconds, 10.0 seconds, etc.

The DP may include two medicament reservoirs, and the **controller** may compare the output value (***Sa***) of the magnetic field sensor to a null value (***Sₙᵤₗₗ***) of the magnetic field sensor to distinguish between engagement of the DP and the RP in a first engagement orientation ('SIDE-B') and engagement of the DP and RP in a second engagement orientation ('SIDE-A'). The controller may compare the output value (***Sa***) of the magnetic field sensor to a first threshold value (***Sth1***) to determine engagement of the DP in the first engagement orientation ('SIDE-B'), and to a second threshold value **(*Sth2*)** to determine engagement in the second engagement orientation ('SIDE-A'), where ***Sth1** > **Sₙᵤₗₗ** > **Sth2.*** The first engagement orientation ('SIDE-B') of the DP is distinguishable from the second engagement orientation ('SIDE-A') of the DP due to (thanks to) the asymmetry of the metal plate with respect to the asymmetry line that coincides with the Z-axis. The controller may output to a user of the pump device (e.g., a patient), audibly and/or visually, an indication regarding engagement between the DP and the RP and/or correctness of the engagement orientation.

The magnet may be a permanent magnet that is configured as a dipole magnet that is magnetized diametrically. The dipole magnet is configured to produce a magnetic field that is parallel to the magnetic field sensing area such that the net magnetic field that is sensed by the magnetic field sensor is zero, or near zero. During engagement of the DP with the RP the net magnetic field sensed by the magnetic field sensor is greater than zero because of the deflection of the magnetic field that the metal plate causes at the magnetic field sensing area.

The magnet may be a permanent magnet that is configured as a multipole magnet. The multipole magnet may include a number ***'n'*** (***n***=1, 2, 3,...) of pairs of conjugated magnetic poles (N/S), and may be magnetized diametrically, or axially, or both diametrically and axially. The multipole magnet is configured to produce multiple magnetic fields in opposite directions at the magnetic field sensing area such that the net magnetic field sensed by the magnetic field sensor is zero, or near zero, due to mutual cancellation of opposing magnetic fields at the magnetic field sensing area. The multipole magnet may be a 4-pole magnet. The 4-pole magnet may include a first pair of conjugate magnetic poles (N/S) that is axially magnetized in a first direction, and a second pair of conjugate magnetic poles (S/N) that is axially magnetized in a second direction opposite the first direction. During engagement of the DP with the RP the magnetic field sensed by the magnetic field sensor is greater than zero due to the metal plate deflecting the magnetic fields at the magnetic field sensing area.

In some embodiments the primary section and the auxiliary section of the asymmetrical metal plate (AMP) are separate, unconnected, sections. In other embodiments the primary section and the auxiliary section of the asymmetrical metal plate (AMP) form one monolithic object.

In some embodiments the primary section of the asymmetrical metal plate (AMP) includes a primary tab that extends inwardly in the AMP, towards the auxiliary section of the AMP. In some embodiments the auxiliary section of the AMP may also include a tab (an auxiliary tab) that extends inwardly in the AMP, towards the primary tab. The primary tab extends inwardly more than the auxiliary tab, and has a greater surface area than the auxiliary tab.

The primary section of the AMP is configured to partially overlap the magnetic field sensing area when the DP and the RP are engaged, with the partial overlapping percentage being ***P***[%]. The value of ***P***[%] is a tradeoff between a magnetic attraction force to be induced between the magnet and the AMP and a magnetic deflection to be induce by the AMP in the magnetic field(s) at the magnetic field sensing area. The value of ***P***[%] may be selected such that the magnetic field sensor does not enter saturation when the DP and the RP are engaged, to enable the controller to distinguish the engagement state from a faulty condition causing the magnetic field sensor to enter saturation. In some embodiments the value of ***P*** is 50% ±10%. The design of the AMP may be a tradeoff between a magnetic attraction force to be induced between the magnet and the asymmetrical metal plate, and a magnetic deflection to be induce by the asymmetrical metal plate in the magnetic field(s) at the magnetic field sensing area.

### BRIEF DESCRIPTION OF THE DRAWINGS

Various exemplary embodiments and aspects are illustrated in the accompanying figures with the intent that these examples be not restrictive. It will be appreciated that for simplicity and clarity of the illustration, elements shown in the figures referenced below are not necessarily drawn to scale. Also, where considered appropriate, reference numerals may be repeated among the figures to indicate like, corresponding or analogous elements. Of the accompanying figures:
Fig. 1 schematically illustrates a cross-sectional view of a magnet-metal plate-sensor ("MMS") setup according to an example embodiment;
Fig. 2A schematically illustrates a diametrically magnetized dipole, circular, flat magnet for producing a magnetic field in a direction parallel to a plane (plane X-Z in Fig. 2A) of a magnetic field sensor, according to an example embodiment;
Fig. 2B shows a diametrically magnetized dipole, rounded, magnet for producing a magnetic field that is similar to the magnetic field shown in Fig. 2A;
Fig. 3A depicts a cross-sectional view of an axially magnetized dipole circular magnet with typical magnetic field;
Fig. 3B depicts a cross-sectional view of a magnet configuration, or layout, including two axially magnetized magnets that produce counteracting magnetic fields that cancel each other's effect at a magnetic field sensor;
Fig. 4A shows an axially magnetized 4-pole magnet for producing two magnetic fields similar to the magnetic fields of Fig. 3B, according to an example embodiment;
Fig. 4B shows a cross-sectional view of the axially magnetized 4-pole magnet of Fig. 4A;
Figs. 5A-5C show a metal plate according to an example embodiment;
Figs. 6A-6B show a metal plate according to another example embodiment;
Figs. 7A-7B show a modification of the metal plate of Figs. 6A-6B according to an example embodiment;
Fig. 8 shows a modification of the metal plate of Figs. 7A-7B, according to an example embodiment;
Fig. 9 depicts various types of metal plate according to other example embodiments;
Figs. 10A-10C depict a metal plate installation in a DP of a pump device according to an example embodiment;
Fig. 11 depicts a metal plate installation in a DP of a pump device according to another example embodiment;
Figs. 12A-12B depict a metal plate installation in a DP of a pump device according to another example embodiment;
Figs. 13A-13B depict a pump device according to an example embodiment;
Fig. 14 depicts a magnet-sensor combination in which a magnetic field sensor is circumferentially surrounded by a magnet, according to an example embodiment;
Figs. 15A and 15B respectively depict a partial length cross-sectional view and a partial width cross-sectional view of a DP and RP of a pump device that are engaged, according to an example embodiment;
Figs. 16A-16B depict an example overlapping between a magnetic field sensor and an asymmetrical metal plate (AMP), according to an example embodiment;
Fig. 17 shows an example output response of a linear Hall effect sensor;
Figs. 18A-18B are comparative figures showing 'saturation' curves vis-a-vis non-saturation curves, according to an example embodiment;
Fig. 19 shows comparative sensor output response curves for two different AMP designs, according to an example embodiment;
Figs. 20A-20B illustrate calibration of an engagement distance according to an example embodiment;
Fig. 21 is a method of determining engagement of a DP and RP of a pump device, according to an example embodiment; and
Fig. 22 is a method of controlling an operation of a pump device according to another example embodiment.

### DETAILED DESCRIPTION OF THE INVENTION

The description that follows provides various details of example embodiments. However, this description is not intended to limit the scope of the claims but instead to explain various principles of the invention and exemplary manners of practicing it.

The description that follows describes a bifunctional magnetic mechanism that is designed to generate a magnetic attraction force to attach a DP of a pump device to a RP of the pump device, and to simultaneously (at the same time) use the same magnetic field to detect engagement of the two parts (DP and RP) of the pump device. The bifunctional magnetic mechanism includes three main parts: (1) a permanent magnet that is configured (in terms of number of pairs of conjugated poles and magnetization) to produce a composite magnetic field from one or more magnetic fields that are orientated in desired direction(s), (2) an asymmetrical metal plate (AMP) that is configured to disrupt the composite magnetic field produced by the magnet, and (3) a magnetic field sensor to sense a level of disruption in the composite magnetic field. The magnet and the magnetic field sensor are incorporated into a RP of a pump device in a way that the magnetic field sensor is circumferentially surrounded by the magnet. The AMP, on the other hand, is incorporated into a DP of the pump device in a way that the AMP subtends the magnetic field sensor (i.e., adjacent to the sensor) when the DP and RP of the pump device are engaged. Disrupting magnetic field(s) by an AMP, which acts as a magnetic shunt, means deflecting (redirecting) some of the magnetic lines of force of the magnetic field(s) from their 'natural', or original, magnetic path.

The AMP is designed to simultaneously perform two types of magnetic interactions with the magnetic field(s) that are produced by the magnet: (1) the AMP is magnetically attractable to the magnet, so that it can magnetically attach the DP and RP of the pump device, and (2) the AMP is also designed to disrupt the magnetic field(s) (e.g., modify or redirect them) that is/are produced by the magnet in a way that would make the disrupted (modified, redirected) magnetic field(s) detectable by the magnetic field sensor. A key point in sensing magnetic field(s) by the magnetic field sensor is that when the two parts intended for attachment (e.g., DP and RP of a pump device) are disengaged (i.e., distanced away from one another), the net magnetic field that the magnetic field sensor senses is zero, or near zero. So, when the net magnetic field that the magnetic field sensor senses is zero, or near zero, a controller, by 'reading' the sensor's output, can determine that the DP and RP of the pump device are not engaged. On the other hand, if the DP and the RP of the pump device are engaged, the disruption caused by the AMP in the magnetic field at, or near, the magnetic field sensor causes the net magnetic field that is sensed by the magnetic field sensor to increase, which facilitates detection of the engagement and disengagement of the two parts (DP, RP) of the pump.

When the DP and the RP are disengaged, the net magnetic field that the sensor senses is zeroed out (or minimized) by one or more magnetic fields that are produced by the magnet in specific direction(s) relative to the sensor's sensing plane (sensing area) of the magnetic field sensor. Producing magnetic field(s) in the required direction(s) by the magnet is obtained by redirecting the magnetic field lines through manipulation of the magnetization scheme of the magnet. Sintered neodymium iron boron magnet (NdFeB) can be multi-pole magnetized according to the needs, i.e., multiple 'N' (north) poles and 'S' (south) poles can be formed, for example, on one plane after magnetization.

### Using magnetic 'shielding' to disrupt (deflect, redirect) magnetic fields

Magnetic shielding generally means surrounding an object (for example a magnetic field sensor) with a magnetically conducting material that can "conduct" magnetic flux better than the materials around it. Using a magnetically conducting material the magnetic field lines tend to 'flow' (i.e., to be redirected) along this material and, thus, avoid the object inside. Using a magnetic shield allows the magnetic field lines to terminate on the opposite poles while giving them a different route to follow. Magnetic flux lines follow a path of least magnetic resistance, which is characterized by having a relatively high magnetic permeability, **µ** (e.g., **µ**> 1.0, where µ=1.0 is the permeability of air). Therefore, if a material with a high magnetic permeability is nearby a magnet, the magnetic flux lines travel the path of least magnetic resistance (through the higher permeability material), leaving less magnetic field in the surrounding air. ('Magnetic permeability' is a scalar quantity quantifying a material's resistance to the magnetic field, or the degree to which magnetic field can penetrate and pass through the material.) A material endowed with highly permeable properties has high magnetic susceptibility to an applied magnetic field; and it readily accepts the flow of magnetic field through it.

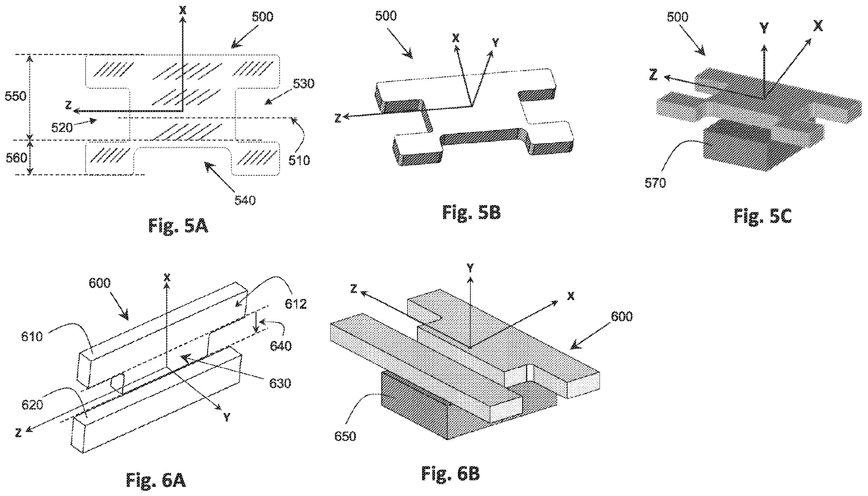

As described herein (for example in connection with Figs. 5A-5C, 6A-6B, 7A-7B, 8, and 9), a metal plate is designed to be asymmetrical with respect to an asymmetry line coinciding with the Z-axis in the drawings in order to disrupt the magnetic field(s) (e.g., deflect magnetic field lines) such that the net magnetic field that is sensed by the magnetic field sensor is no longer zero when the DP and RP of the pump device are engaged. In a way, the AMP functions as a shielding means that unbalances the zero net magnetic field at the magnetic field sensor by deflecting, or redirecting, at least some of the magnetic field lines in the space surrounding the sensor. It is the change in direction of magnetic field lines that causes the net magnetic field to increase.

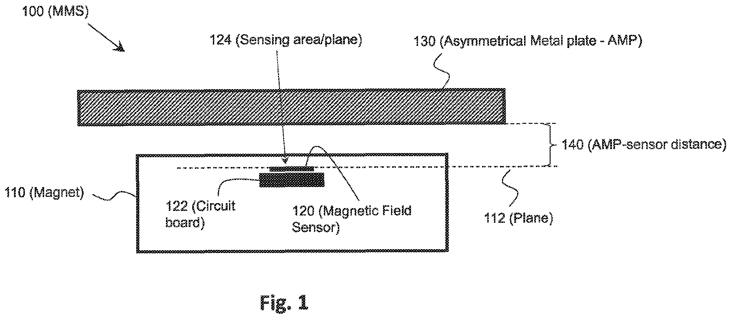

Fig. 1 schematically illustrates a cross-sectional view of a magnet-metal plate-sensor (MMS) setup **100** according to an example embodiment. MMS **100** includes a magnet **110** (for example a permanent magnet), a magnetic field sensor **120,** a circuit board **122** for powering magnetic field sensor **120** and for converting an analog output signal (e.g., output voltage) of magnetic field sensor **120** to corresponding digital signal, and an asymmetrical metal plate (AMP) **130.** Magnet **110** is generally flat and coincides with a plane **112.** Magnetic field sensor **120** has a magnetic field sensing area/plane **124** that is parallel, or generally parallel, to plane **112.**

Magnet **110** circumferentially surrounds magnetic field sensor **120** and is magnetized to produce one or more magnetic fields in particular direction(s) to zero-out a net magnetic field that is sensed by magnetic field sensor **120** when AMP **130** and sensor **120** are distanced away from one another.

Asymmetrical metal plate (AMP) **130** is configured to be magnetically attracted to magnet **110** when AMP **130** and magnet **110** are positioned adjacent to each other, and, in addition, to disrupt (e.g., deflect) the one or more magnetic fields at the magnetic field sensing area **124** when AMP **130** is adjacent to magnetic field sensing area **124** (e.g., at detection distance **140** from magnetic field sensing area **124,** or closer), to thereby increase the net magnetic field that is sensed by magnetic field sensor **120.**

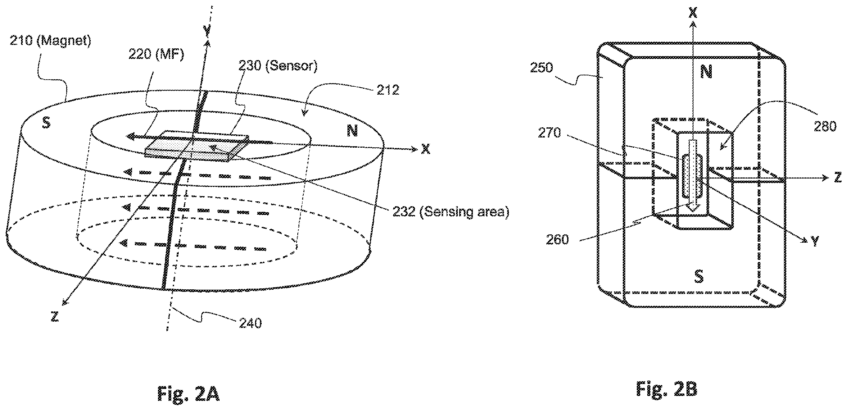

Fig. 2A schematically illustrates a diametrically magnetized dipole, circular, flat magnet **210** (e.g., a permanent magnet) for producing a magnetic field **220** in a direction that is parallel to a sensing plane (plane X-Z in Fig. 2A) of a magnetic field sensor **230,** according to an example embodiment. Magnet **210** has an axis **240** that coincides with the Y-axis. As illustrated in Fig. 2A, magnetic field sensor **230,** which is a flat sensor having a magnetic field sensing area **232**, is slightly (e.g., a few millimeters) 'sunk' (indented with respect to the surface **212** of magnet **210)** in the center of magnet **210,** and circumferentially surrounded by the magnet.

Dipole magnet **210** is a ring-shaped magnet including a North ('N') pole and a South ('S') pole opposite the N pole. Using this MMS layout/configuration, the direction of magnetic field **220,** if undisrupted, is parallel to the sensor's sensing area/plane **232** of magnetic field sensor **230,** meaning that the angle between the sensor's magnetic sensing area and the magnetic field lines is zero degrees. Therefore, the net magnetic field that magnetic field sensor **230** senses is zero. However, when an AMP (example AMPs are shown, for example, in Figs. 5A-5C, 6A-6B, 7A-7B, 8, 9, 10B-10C, and 12A-12B) is brought close enough to the magnetic field sensing area **232** of sensor **230,** the magnetic field lines are disrupted (redirected) in a way that the angle between the sensor's magnetic sensing area **232** and at least some of the redirected magnetic field lines is no longer zero, which makes them detectable by magnetic field sensor **230.** Preferably, the extent to which the AMP should disrupt the magnetic field lines, and thus make them readily detectable by sensor **230,** may be predetermined based on, for example, the following factors: (1) the sensor's output dynamic range (e.g., the sensor's operational output dynamic range, as opposed to the sensor's potential full output dynamic range, (2) the engagement distance between the sensor and the AMP, and (3) the maximal detectable distance between the sensor and the AMP. As described herein, the AMP is a bifunctional metal plate, where the second function is producing a magnetic attraction force that is strong enough to magnetically attach (releasably attach) two devices together, for example DP and RP of a pump device. Accordingly, the magnetic attraction force is another factor to consider when designing an AMP.

Fig. 2B shows a diametrically magnetized dipole, rounded, magnet **250,** which is analogous to magnet **210** of Fig. 2A, for producing a magnetic field **260** that is analogous to magnetic field **220** of Fig. 2A. Fig. 2B shows an example implementation of the magnetic field sensing principle described in connection with Fig. 2A. In Fig. 2B, magnetic field sensor **270** is similar to magnetic field sensor **230** of Fig. 2A. Magnet **250** includes a central aperture **280,** and magnetic sensor **270** is positioned inside aperture **280** such that magnetic sensor **270** is centered in the middle of magnet **250,** so it is circumferentially surrounded by magnet **250.**

Like in Fig. 2A, the direction of the magnetic field **260,** if undisrupted, is parallel to the sensing area/plane of magnetic field sensor **270** (the angle between the sensor's magnetic sensing area/plane and the magnetic field lines is zero degrees). Therefore, the net magnetic field that magnetic field sensor **270** senses is zero. However, when an AMP (example AMPs are shown, for example, in Figs. 5A-5C, 6A-6B, 7A-7B, 8, 9, 10B-10C, and 12A-12B) is brought close enough to the magnetic field sensing area of sensor **270,** the magnetic field lines are disrupted (redirected) in a way that the angle between the sensor's magnetic sensing area and at least some of the redirected magnetic field lines is no longer zero, which makes them detectable by magnetic field sensor **270.**

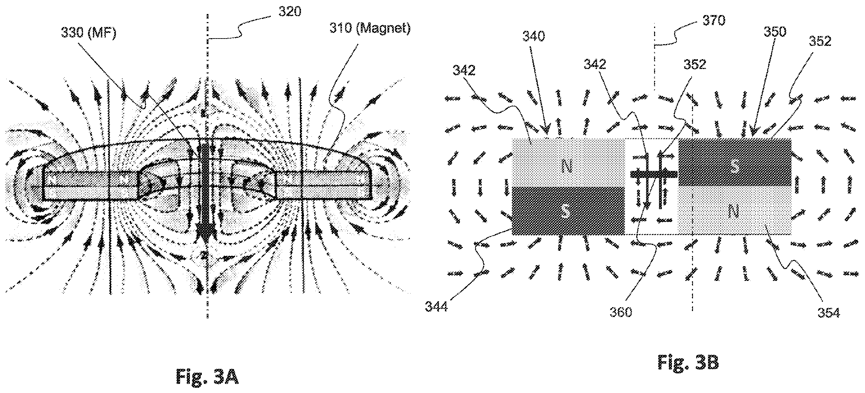

Fig. 3A depicts a cross-sectional view of an axially magnetized dipole magnet **310** with typical magnetic field. Magnet **310** is a dipole, ring like, magnet that is axially magnetized. Namely, magnet **310** is magnetized in a direction of the magnet's axis **320,** with the 'N' pole being, in this example, the upper pole. Given the magnetization configuration of ring magnet **310,** magnet **310** produces a relatively uniform magnetic field **330** in the center area of magnet **310.** In the center area of magnet **310** the direction of magnetic field **330** is vertical from the 'N' pole to the 'S' pole, and, therefore, magnetic field **330** coincides with the axis **320** of magnet **310.**

Fig. 3A illustrates generation of a magnetic field in the center area of magnet **310.** If a magnetic field sensor is situated in the center area of magnet **310** in a way that its sensing plane is perpendicular to axis **320,** the sensor would always detect a magnetic field, which is a drawback in terms of the ability to use this configuration to magnetically detect an engagement between two parts/devices, for example between a DP and RP of the pump device. It would be beneficial (in terms of engagement detection) to produce a second magnetic field that would counteract (cancel out) the effect of magnetic field **330** at the magnetic field sensor at the center of the magnet when the two magnetic fields are free of magnetic disruption. The basic principle of active cancellation of the effect of two magnetic fields at a magnetic field sensor situated in the center of a magnet is shown in Fig. 3B, which is described herein below. Briefly, one magnetic field is produced in a first direction by a first pair of magnetic poles, while the cancelling (counteracting) magnetic field is produced in the opposite (counteracting) direction by a second pair of magnetic poles.

Fig. 3B depicts a cross-sectional view of a magnet configuration including two axially magnetized magnets **(340, 350)** that produce two, counteracting, magnetic fields that cancel each other's effect at a magnetic field sensor **360.** Each of axially magnetized magnets **340** and **350** produces a typical magnetic field, and the two magnets also magnetically interact with one another because each 'N' pole interacts with two 'S' poles. The vector directions of the magnetic fields that are produced by the two magnets are shown in Fig. 3B.

The two magnetic fields that the two, adjacent, magnets **(340, 350)** produce in the space between them (i.e., in the space shared by them) are relatively uniform and antiparallel (i.e., they are directed to opposite directions). This phenomenon is obtained by using pairs of N/S poles that produce magnetic fields in opposite directions. For example, axially magnetized magnet **340** includes a first pair of conjugated N/S poles that produces a magnetic field **342** in a first direction coinciding with 'symmetry' line **370,** and axially magnetized magnet **350** includes a second pair of conjugated S/N poles that produces a magnetic field **352** in a second direction that also coincides with symmetry line **370.** However, magnetic field **342** and magnetic field **352** are directed to opposite directions. (Magnetic field **342** and magnetic field **352** are antiparallel vector fields.) North pole **342** and south pole **344** make up a first pair of conjugated poles, and south pole **352** and north pole **354** make up a second pair of conjugated poles.

Axially magnetized magnets **340** and **350** are designed such that they produce magnetic fields with the same magnetic field strength at a same point, or area, in a space between the two magnets, and the two magnets direct the two magnetic fields to opposite directions at that point, or area, in order for them to cancel out each other at that point or area. The point (or area) where magnetic fields cancel out each other is called 'neutral point' (or 'neutral area'). This kind of magnetic field cancellation occurs because magnetic fields obey the superposition principle, so two magnetic fields that are opposite in directions are subtracted, and if the two magnetic fields are equal in strength, subtracting them results in a zero magnetic field at that point or area. So, if magnetic fields **342** and **352** have equal magnitude, and given their opposite directions, the net magnetic field that magnetic field sensor **360** senses (detects) at the neutral point/area is zero.

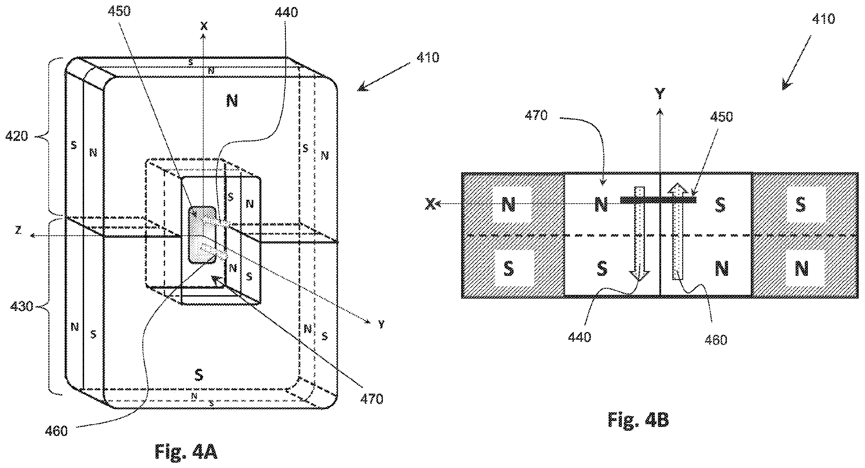

Like in Figs. 2A-2B, if neither of magnetic fields **342** and **352** is disrupted the net magnetic field that magnetic field sensor **360** senses is zero because sensor **360** is situated at the neutral point, where the antiparallel magnetic fields **342** and **352** counteract each other. However, if an AMP (not shown in Fig. 3B) is brought near the neutral point (near the magnetic field sensing area of sensor **360),** magnetic fields **342** and **352** no longer cancel out each other because of uneven disruption that the AMP induces in the two magnetic fields. Unevenly disrupting the two magnetic fields makes them detectable by magnetic field sensor **360.** The discussion above related to the AMP is also applicable to Fig. 2B and to Fig. 4A.

Fig. 4A shows an axially magnetized 4-pole magnet **410** for producing two magnetic fields that are analogous to magnetic fields **342** and **352** of Fig. 3B. Magnet **410** (an example special case of a multipole magnet) is an example implementation of the super position principle described in connection with Fig. 3B. Rounded (ring-like) magnet **410** has an axis that coincides with the Y-axis and includes a first part **420** forming a first pair of conjugated N/S poles that is axially magnetized to produce a first magnetic field **440** in a first direction at the center area of magnet **410** (at magnetic field sensor **450),** and a second part **430** forming a second pair of conjugate N/S poles that is axially magnetized to produce a second magnetic field **460** in a second direction at the center area of magnet **410** (at magnetic field sensor **450),** which is opposite the direction of magnetic field **440.** Like in Fig. 3B, axially magnetized magnet **410** produces two counteracting magnetic fields **(440, 460)** of equal strength that cancel each other at magnetic field sensor **450** by virtue of superposition of the two equal and antiparallel magnetic fields. (Magnetic field **440** and magnetic field **460** are antiparallel vector fields.) Fig. 4B shows a length cross-sectional view of the axially magnetized 4-pole magnet **410** of Fig. 4A. Magnet **410** includes a central aperture **470,** and magnetic sensor **450** is positioned inside aperture **470** such that magnetic sensor **450** lies in the X-Y plane and is centered in the middle of magnet **410,** so it is circumferentially surrounded by magnet **410.**

An axially magnetized magnet (e.g., 4-pole magnet **410,** Figs. 4A-4B) can produce a relatively high density of magnetic field flux lines in the Y-axis direction in the drawings. This means that the magnetic attraction force induced between an AMP and the magnet during attachment of the DP and RP of the pump device is greater in the 4-pole magnet comparing to the magnetic attraction force that is induced by a dipole magnet.

Figs. 5A-5C show an asymmetrical metal plate (AMP) **500** according to an example embodiment. Like all AMPs, AMP **500** is flat and has an asymmetry line **510.** (AMP **500** is asymmetrical with respect to asymmetry line **510** that coincides with the Z-axis of the Cartesian coordinate system.) AMP **500** is a monolithic object having a 'flatness plane' that, like in other AMPs, coincides with the X-Z plane of the cartesian coordinate system.

Referring to Fig. 5A, AMP **500** includes three peripheral recesses: two side recesses **520** and **530** that, in this example, are symmetrical with respect to the X-axis, and one 'frontal' recess **540** that, in this example, is parallel to the Z-axis. Side recesses **520** and **530** do not necessarily have to be symmetrical with respect to the X-axis, nor does recess **540** have to be parallel to the Z-axis or symmetrical with respect to the X-axis. For example, recesses **520, 530** and **540** may have different size, shape, width and/or recess depth (indentation), and they may generally be inclined with respect to the X-axis or Z-axis. Recess **540** does not necessarily have to be symmetrical with respect to the X-axis. Recess **540** causes asymmetry with respect to, or about, the Z-axis (i.e., there is no recess on the opposite side of AMP **500)** and in the direction of the X-axis, thus making AMP **500** an asymmetrical metal plate (AMP). Imparting asymmetry to an AMP (e.g., AMP **500)** with respect to, or about, the Z-axis is beneficial in terms of the impact a magnetic disruption induced by the AMP has on the magnetic field(s) involved, hence on the net magnetic field that can be detected by a magnetic field sensor (e.g., sensor **450,** Figs. 4A-4B). (The asymmetry imparted to the AMP with respect to, or about, the Z-axis unevenly disrupts the magnetic field(s), for example magnetic fields **440** and **460,** causing the net magnetic field to be detectably greater than zero.)

Structurally, AMP **500** includes a first (primary) section **550** that is configured to disrupt (modify) the magnetic field(s) produced by the magnet (e.g., magnet **110, 250,** or **410)** when AMP **500** is at, or near, the magnet (hence at or near the magnetic field sensor that is surrounded by the magnet). The extent to which the magnetic field(s) produced by the magnet are disrupted (modified, distorted) by AMP **500** primarily depends on the size, shape and material of section **550.** In general, the greater the area of first section **550,** the greater the disruption to the magnetic field(s), hence the output signal of the magnetic field sensor **570** (Fig. 5C). However, since the sensor's dynamic output range is limited (it depends, among other things, on the electrical design of the related electrical circuit), a trade off needs to be made between the design of first section **550** and the saturation values of the sensor's output. First section **550** may be designed to accommodate for a limited dynamic output range of the sensor to avoid saturation state. Section **550** is also configured to induce, simultaneously to disrupting the magnetic fields, a magnetic attraction force between the magnet and section **550.** In general, the greater the area of first section **550,** the greater the magnetic attraction force that is induced between first section **550** and the magnet. Therefore, the design of section **550** is also a tradeoff between the dynamic range of the sensor's output and the strength of the resulting magnetic attraction force.

AMP **500** also includes a second (an auxiliary) section, that is section **560,** that is designed to produce an additional magnetic attraction force to thereby increase the overall magnetic attraction force that is induced between the magnet and AMP **500.** (Section **560** of AMP **500** plays a negligible role, if at all, in the disruption of the magnetic field(s).)

Fig. 5B shows a three-dimensional view of AMP **500,** and Fig. 5C shows AMP **500** in close proximity to a magnetic field sensor **570.** (The close proximity between AMP **500** and magnetic field sensor **570** signifies, or represents, proper attachment, or engagement, between an element or part that includes AMP **500** (e.g., a DP of a pump device), and an element or part that includes magnetic field sensor **570** (e.g., RP of the pump device).)

Figs. 6A-6B show three-dimensional views of an asymmetrical metal plate (AMP) **600** according to another example embodiment. AMP **600** is a 2-part metal plate including a first (primary) part **610** and a separate second (auxiliary) part **620.** First part **610** functions in a similar way as first section **550** of Fig. 5A, meaning that part **610** is configured to: (1) disrupt (distort, modify) magnetic field(s) that are produced by the magnet (e.g., magnet **110, 250,** or **410)** when AMP **600** is at, or near, the magnet (hence at or near the magnetic field sensor surrounded by the magnet), and (2) produce, simultaneously to disrupting the magnetic field(s), a magnetic attraction force between the magnet and first part **610** of AMP **600.**

Second part **620** functions in a similar way as second (auxiliary) section **560** of Fig. 5A, meaning that part **620** is configured to produce an additional magnetic attraction force to thereby increase the overall magnetic attraction force that is induced between the magnet and part **620** of AMP **600.** (Part **620** of AMP **600** plays a negligible role, if at all, in disrupting the magnetic field(s).)

Part **610** includes a peripheral base **612** and a tab **630** that extends/protrudes **(640)** from peripheral base **612** inwardly, along the X-axis, towards auxiliary part **620.** Tab **630** imparts asymmetry to AMP **600** with respect to the Z-axis. The size and shape of tab **630** may differ, or deviate, from those that are shown in Fig. 6A, so long as the asymmetry of AMP **600** with respect to the Z-axis is maintained. (As described herein, asymmetry of an AMP with respect to the Z-axis is beneficial in terms of the uneven disruption that the AMP induces in the magnetic field(s) that are produced by the magnet. An AMP can be designed in any way, provided that the design conforms to the 'uneven magnetic disruption' principle disclosed herein.) AMP **600** is shown symmetrical with respect to the X-axis, though this is not a prerequisite for proper functioning of AMP **600.**

Fig. 6B shows AMP **600** adjacent to a magnetic field sensor **650.** The close proximity between AMP **600** and magnetic field sensor **650** signifies, or represents, attachment, or engagement, between an element or part that includes AMP **600** (e.g., DP of a pump device), and an element or part that includes magnetic field sensor **650** (e.g., RP of the pump device). Like in the case of AMP **500,** the design of AMP **600** is a tradeoff between the size and shape of parts **610** and **620** of AMP **600,** the output dynamic range of the sensor, and the strength of the magnetic attraction force that is induced between the magnet and AMP 600 as a whole.

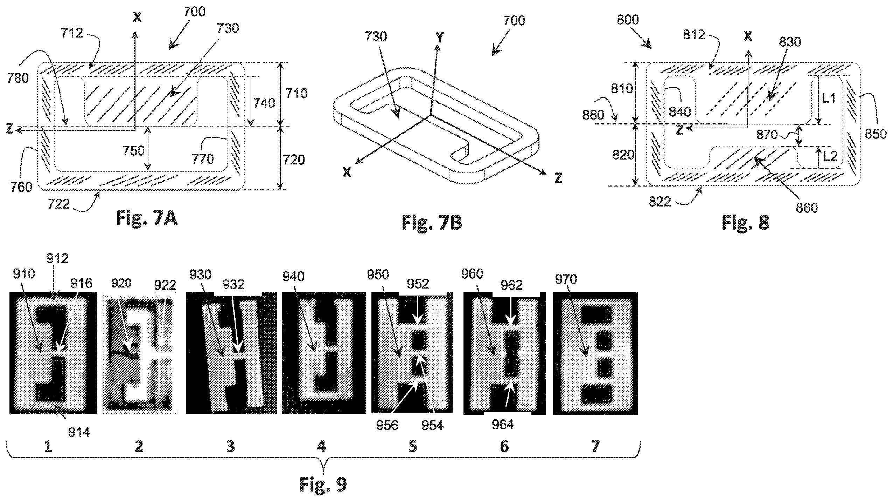

Figs. 7A-7B show an AMP (AMP **700)** that is a modification of AMP **600** of Figs. 6A-6B according to an example embodiment. (Fig. 7B shows a three-dimensional view of AMP **700.)** AMP **700** is a 2-section AMP including a first (primary) section **710** and a second (auxiliary) section **720.** Primary section **710** functions in a similar way as primary part **610** of Figs. 6A-6B. Namely, section **710** is configured to: (1) disrupt (distort, modify) magnetic field(s) that are produced by the magnet (e.g., magnet **110, 250,** or **410)** when AMP **700** is at, or near, the magnet (hence at or near the magnetic field sensor), and (2) produce, simultaneously to disrupting the magnetic field(s), a magnetic attraction force between the magnet and first section **710** of AMP **700.**

Section **720** of AMP **700** functions in a similar way as auxiliary part **620.** Namely, section **720** is configured to produce an additional magnetic attraction force to increase the overall magnetic attraction force that is induced between the magnet and AMP **700.** (Auxiliary section **720** of AMP **700** plays a negligible role, if at all, in the disruption of the magnetic field(s).)

Section **710** includes a peripheral base **712** and a tab **730** (primary tab) that extends/protrudes **(740)** from peripheral base **712** inwardly, along the X-axis, towards auxiliary section **720.** An air gap **750** exists between tab **730** and a peripheral base **722** of auxiliary section **720.** Tab **730** imparts asymmetry to AMP **700** with respect to the Z-axis, with line **780** being the asymmetry line between section **710** and section **720.** The size and shape of tab **730** may differ, or deviate, from those that are shown in Figs. 7A-7B, so long as the asymmetry of AMP **700** with respect to asymmetry line **780** (and the Z-axis) is maintained. Tab **730** (and AMP **700** as a whole) is symmetrical with respect to the X-axis, though this is not a necessary condition for proper, or acceptable, functioning of AMP **700.**

AMP **700** differs from AMP **600** of Figs. 6A-6B in that primary section **710** and auxiliary section **720** are structurally interconnected by connecting elements (peripheral elements) **760** and **770** that make AMP **700** a closed rounded flat object. Interconnecting primary section **710** and auxiliary section **720** by peripheral interconnecting elements **760** and **770** is beneficial in terms of manufacturing and assembly processes. Similarly to AMPs **500** and **600,** the design of AMP **700** is a tradeoff between the size and shape of sections **710** and **720** of AMP **700,** the material(s) the AMP is made of, the output dynamic range of the magnetic field sensor, and the strength of the magnetic attraction force that is induced between the magnet and AMP **700** as a whole.

Fig. 8 shows an AMP **800** that is an example modification of AMP **700** of Figs. 7A-7B, according to an example embodiment. AMP **800** is a 2-section AMP including a first (primary) section **810** and a second (auxiliary) section **820.** Similarly to AMP **700,** primary section **810** of AMP **800** includes a peripheral base **812** and a first tab **830** (a primary tab) that extends/protrudes **(L1)** inwardly, in the direction of the X-axis, from peripheral base **812** towards auxiliary section **820.** AMP **800** is also similar to AMP **700** of Figs. 7A-7B in that primary section **810** and auxiliary section **820** are structurally interconnected by peripheral interconnecting elements **840** and **850.** Similarly to AMP **700,** interconnecting primary section **810** and auxiliary section **820** by peripheral interconnecting elements **840** and **850** is beneficial in terms of manufacturing and assembly processes.

AMP **800** differs from AMP **700** in that AMP **800** includes a second (an auxiliary) tab **860.** Auxiliary tab **860** extends/protrudes **(L2)** inwardly, in the direction of the X-axis, from a peripheral base **822** of auxiliary section **820** towards first tab **830,** leaving an air gap **870** between 'primary' tab **830** and 'auxiliary' tab **860.** Protrusion length **L1** of tab **830** of primary section **810** is greater than the protrusion length **L2** of tab **860** of auxiliary section **810 (L1>L2).**

Primary section **810** functions in a similar way as primary section **710** of Figs. 7A-7B. Namely, primary section **810** is configured to: (1) disrupt (distort, modify) magnetic field(s) that are produced by the magnet (e.g., magnet **110, 250,** or **410)** when AMP **800** is at, or near, the magnet (hence at or near the magnetic field sensor), and (2) induce, simultaneously to disrupting the magnetic field(s), a magnetic attraction force between the magnet and primary section **810** of AMP **800.**

Auxiliary section **820** functions in a similar way as auxiliary part **720.** Namely, auxiliary section **820** is configured to produce an additional magnetic attraction force to increase the overall magnetic attraction force that is induced between the magnet and AMP **800.** (Auxiliary section **820** of AMP **800** plays a negligible role, if at all, in the disruption of the magnetic field(s).) Most of the additional magnetic attraction force that is provided by auxiliary section **820** is provided by tab **860.**

The size (width and/or length) and shape of air gap **870** and its location along the X-axis are designed as, or embody, a tradeoff between the intended magnetic disruption that AMT **800** is to induce in the magnetic fields produced by the magnet (hence the resulting output dynamic range of the magnetic field sensor), and the magnetic attraction force that AMT 800 induces vis-à-vis the magnet.

The design of tabs **830** and **860** (including size and shape) imparts asymmetry to AMP **800** with respect to the Z-axis, with line **880** being the asymmetry line between section **810** and section **820.** The size and shape of tabs **830** and **860** may differ, or deviate, from those that are shown in Fig. 8 so long as the asymmetry of AMP **800** with respect to the Z-axis is maintained. Tabs **830** and **860** (and AMP **800** as a whole) are symmetrical with respect to the X-axis, though this is not necessary for proper, or acceptable, functioning of AMP **800.**

Fig. 9 depicts various AMP designs according to some example embodiments. The seven AMPs of Fig. 9 are variants of AMT **700** Figs. 7A-7B, and each of these variants includes one tab similar to tab **730** of Figs. 7A-7B: tab **910** in Fig. 9(1), tab **920** in Fig. 9(2), tab **930** in Fig. 9(3), tab **940** in Fig. 9(4), tab **950** in Fig. 9(5), tab **960** in Fig. 9(6), and tab **970** in Fig. 9(7).

In the description that follows 'Fig. 9(1)' means "Fig. 9, variant No. 1"; 'Fig. 9(2)' means "Fig. 9, variant No. 2", and so on:
- Fig. 9(1) shows an AMP that, similarly to Figs. 7A-7B, includes two peripheral connecting elements **912** and **914** that interconnect a primary section of the AMP and an auxiliary section of the AMP. The AMP also includes a middle 'bridge' connector (strip) **916** that also interconnects the AMP's primary section and auxiliary section;
- Fig. 9(2) shows an AMP that includes two peripheral connecting elements that are similar to peripheral connecting elements **912** and **914** of Fig. 9(1), but that lacks bridge connector **916.** Instead of having a bridge connector, the AMP of Fig. 9(1) includes an air gap **922;**
- Fig. 9(3) shows an AMP that includes a bridge connector **932** similar to bridge connector **916** of Fig. 9(1), but that lacks the two peripheral connecting elements;
- Fig. 9(4) shows an AMP that includes a bridge connector and a peripheral connecting element that are respectively similar to bridge connector **916** and peripheral connecting element **914** of Fig. 9(1), but that lacks the other peripheral connecting element **(912)** of Fig. 9(1);
- Fig. 9(5) shows an AMP that includes three bridge connectors **(952, 954** and **956),** but that lacks the two peripheral connecting elements **(912, 914)** of Fig. 9(1);
- Fig. 9(6) shows an AMP that resembles the AMP of Fig. 9(5). The AMP of Fig. 9(6) includes two of the three bridge connectors of Fig. 9(5) (i.e., bridge connectors **962** and **964),** but it lacks the two peripheral connecting elements **(912, 914)** and middle bridge connector **(954)** of Fig. 9(5), and
- Fig. 9(7) shows an AMP that is a combination of the AMP of Fig. 9(1) and the AMP of Fig. 9(5). Namely, the AMP of Fig. 9(7) includes two peripheral connecting elements similar to peripheral connecting elements **912** and **914** of Fig. 9(1), and, in addition, three bridge connectors similar to bridge connectors **952, 954** and **956** of Fig. 9(5).

The bridge connectors shown in Figs. 9(1) and 9(2)-9(7) are only structural elements interconnecting the primary sections of the AMPs and the auxiliary sections of the AMPs. Therefore, a bridge connector can structurally be made as narrow as possible (a bridge connector may be a few millimeters wide) and have an area that is very small comparing to the area of the AMP's tab. The magnetic field disruption and magnetic attraction force that a bridge connector induces are, therefore, negligible.

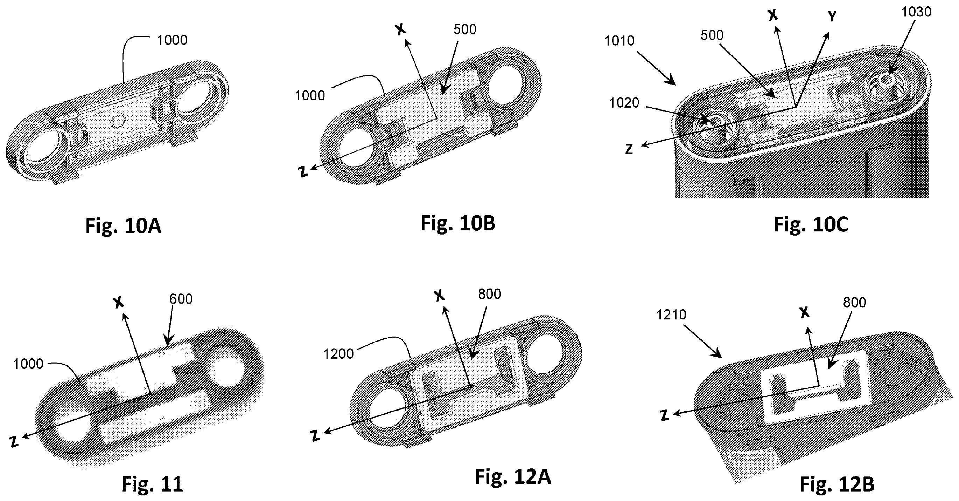

Figs. 10A-10C depict stages of installation of an AMP **500** in a DP **1010** of a pump device according to an example embodiment. Fig. 10A depicts a housing top cover **1000** of a DP of a pump device that is designed to accommodate a flat AMP such as, for example, AMP **500** of Figs. 5A-5C, or a similar AMP. Fig. 10B depicts AMP **500** (an example AMP) that is securely seated in housing top cover **1000.** Fig. 10C depicts a 2-reservoir DP **1010** of a pump device. All DPs that are shown in the drawings and/or described herein, including DP **1010,** are manufactured by using plastic injection molding. A DP that is made of plastic does not interfere with the magnetic interactions between the AMP and the magnet, or with the magnetic interaction between the AMP and the magnetic field sensor. (Plastic materials are 'transparent' to magnetic fields.)

The assembly process of DP **1010** includes, among other things, attaching housing top cover **1000** (with AMP **500** seating therein) to DP **1010.** In this example, DP **1010** includes two medicament reservoirs, one of which is accessible (operable) via luer connector **1020,** and the other reservoir is accessible (operable) via luer connector **1030.**

Fig. 11 depicts a different AMP (e.g., AMP **600)** that is securely seating inside housing top cover **1000.** ('Reservoir accessible via a luer connector' means that the luer connector enables the reservoir to be filled up with medication during preparation of the pump device for operation, and, then, emptied when medicament is expelled out of the reservoirs during treatment.)

Figs. 12A-12B depict an installation of AMP **800** in a DP **1210** of a pump according to an example embodiment. Fig. 12A depicts AMP **800** of Fig. 8 securely seating in housing cover **1200.** Fig. 12B depicts a medicament DP **1210** of a pump device. The assembly process of DP **1210** includes attaching housing cover **1200** (with AMP **800** seating therein) to DP **1210.** Similarly to DP **1010** of Fig. 10C, DP **1210** also includes two medicament reservoirs, where each reservoir is accessible (operable) via a luer connector. (A DP may include only one reservoir.)

The AMP (e.g., AMP **500** in Fig. 10B, AMP **600** in Fig. 11, AMP **800** in Fig. 12A) may be secured to the housing cover (e.g., to housing top cover **1000** in Fig. 10B and housing top cover **1200** in Fig. 12A) by swaging, wherein the AMP is pressed or forced into the surface of the housing cover. In a particular embodiment, the AMP is sintered or hot-pressed into the housing cover. In other embodiments a cold swaging procedure is employed to secure the AMP to the housing cover. In other embodiments the AMP is secured to the housing cover by mechanically fitting the AMP in a depression that is formed in the housing cover, and the AMP has a size and shape that match the size and shape of the depression in the housing cover.

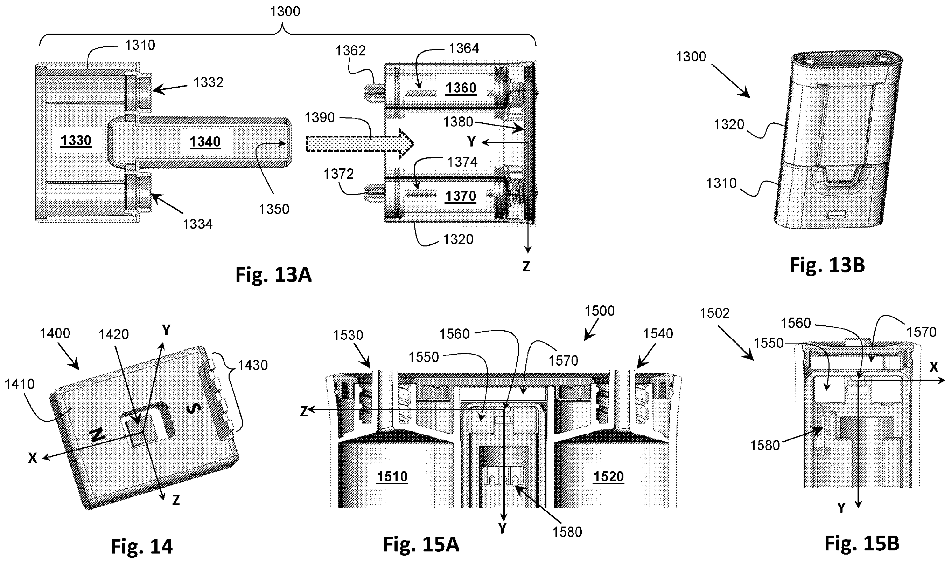

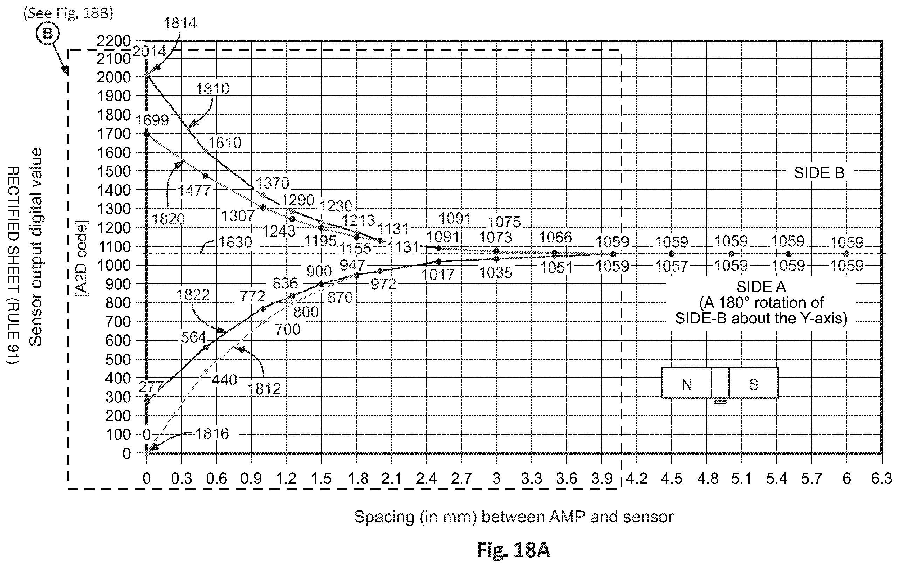

Figs. 13A-13B depict an example pump device **(1300)** including a RP **(1310)** and a DP (**1320**) according to an example embodiment. Referring to Fig. 13A, by way of example RP **1310** has a "T-shaped" configuration including a main body **1330,** and a middle 'leg' portion **1340** that protrudes (extends out) from the pump's main body **1330.** Main body **1330** contains, among other things, an electrical circuit board, a controller, a gear unit for driving two plunger heads in DP **1320,** two 'sleeves' for accommodating two drive screws that respectively move the two plunger heads, etc. Middle leg portion **1340** contains, among other things, an electric motor for driving the gear unit, and a magnet-sensor combination. The magnet-sensor combination includes a magnet that is configured to produce one or more magnetic fields in preselected direction(s), and a magnetic field sensor for sensing a disruption in the magnetic field(s) when DP **1320** is engaged with RP **1310.** In addition to producing the magnetic fields by the magnet, the magnet is also used to magnetically attract an asymmetrical metal plate (AMP). The magnet and the magnetic field sensor (both elements are not shown in Figs. 13A-13B) are positioned at the distal end **1350** of middle leg portion **1340.** The magnet is formed in a suitable size and shape to be accommodated in (received by) the housing of middle leg portion **1340,** and is configured to provide a magnetic field with a pattern, strength, and direction(s) that, in conjunction with the used AMP, would enable the pump's controller to distinguish between 'engagement' state (in which the DP and RP of the pump device are engaged) and 'disengagement' state (in which the DP and RP of the pump device are disengaged) based on the output value of the magnetic field sensor. (The way the controller distinguishes between the 'engagement' state and the 'disengagement' state of the pump device is described, for example, in connection Fig. 18A-18B.)

Disposable part **1320** of pump device **1300** may generally include one or more medicament reservoirs. By way of example, DP **1320** includes two medicament reservoirs (reservoirs **1360** and **1370).** Disposable part **1320** also includes an AMP **1380** to magnetically interact with the magnet-sensor combination at **1350** when RP **1310** and DP **1320** are engaged **(1390).** During engagement **(1390),** the distance (air gap) between AMP **1380** in the DP and the magnet-sensor combination at **1350** in the RP is getting shorter, leading to increased disruption in the magnetic field(s) that is/are sensed by the magnetic field sensor at **1350.** Increasing the magnetic disruption by AMP **1380** has bearing on the value of the sensor's output in a way that enables the controller in RP **1310** to determine whether RP **1310** and DP **1320** are engaged or disengaged. During engagement of RP **1310** and DP **1320** a driving nut **1362** associated with reservoir **1360** is engaged with, and rotatable by, a driving gear at **1332** to linearly move a driving screw **1364,** hence a plunger head in reservoir **1360.** Similarly, a driving nut **1372** associated with reservoir **1360** is engaged with, and rotatable by, a drive gear at **1334** to linearly move a driving screw **1374,** hence a plunger head in reservoir **1370.**

Fig. 13B depicts RP **1310** and DP **1320** in the engagement state. In the engagement state, AMP **1380** is the closest to the magnet at **1350.** (In the engagement state the distance between AMP **1380** and the magnet at **1350** is zero, or near zero, as AMT **1380** is magnetically attached to the magnet.) This means that the distance (air gap) between AMP **1380** and the magnetic field sensor at **1350** is minimal (but not zero), leading to the sensor outputting its maximal output value per the design specifics of the used AMT and sensor. As Figs. 4A-4B (for example) show, magnetic field sensor **450** is slightly sunk in magnet **410** (e.g., slightly indented relative to the surface of the magnet) and circumferentially surrounded by magnet **410.** This is the reason why the distance between the AMP and the sensor is not zero when RP **1310** and DP **1320** are engaged. However, in other designs or embodiments, the sensor's sensing area and the magnet's outer surface may lie in the same plane, in which case the engagement distance between the sensor and the AMP can be zero.

AMP **1380** may resemble AMP **500,** AMP **600,** AMP **700,** or AMP **800,** or it may have a different design so long as the design of the AMP simultaneously complies with the two requirements described herein; namely, the AMP can cause a sensor-detectable magnetic disruption in the magnetic field(s) that is(are) produced by the magnet, and it can induce a sufficiently strong magnetic attraction force between the AMP and the magnet to secure engagement between the RP and DP of the pump device. Common to all AMP designs is the notion that the magnetic attraction force induced between the AMP and the magnet should not be too strong in order to enable a user, for example a PD subject, to effortlessly disengage the two parts.

### Detecting pump device engagement and engagement orientation from the sensor's output

Fig. 13A, for example, shows a 2-reservoir DP **1320** of a pump device that is symmetrical with respect to middle leg portion **1340** of RP **1310** of pump device **1300.** This symmetricity enables DP **1320** to engage RP **1310** in a first engagement orientation, as shown in Fig. 13A (reservoir **1360** is upper in the drawing), or in a second engagement orientation (in which reservoir **1360** is 'below' reservoir **1370),** where the second engagement orientation that is obtained by rotating DP **1320** 180 degrees about the Y-axis.

In some embodiments, both reservoirs (e.g., reservoirs **1360** and **1370** of Fig. 13A) contain the same medicament, so the question *"Which orientation should be used?"* is immaterial therapeutically because the two orientations are interchangeable (i.e., they can likewise be used to obtain the same therapy result). However, in other embodiments, each of reservoir **1360** and reservoir **1370** may contain a different type of medicament, so the question *"Which orientation should be used?"* may be of great importance therapeutically because applying a specific drug regimen may necessitate a specific engagement orientation of the DP. In these embodiments, the pump's controller may be configured to control the two reservoirs individually and independently according to the therapy requirements, for example to deliver to the patient an intended amount of medicament #1 from reservoir **1360** (for example), and, thereafter, delivering an intended amount of medicament #2 from reservoir **1370.** So, in such cases, the controller has to 'make sure' that the engagement orientation of the DP suits the intended treatment regimen before the controller operates the pump device to deliver the two medicaments.

So, as described herein, detecting only an engagement between a DP and a RP of a pump device may not suffice for normal operation of the pump device because, depending on the type of medicament in each reservoir and/or medicament regimen, the engagement orientation may also be of importance, hence needs to be checked for correctness. Therefore, the controller may be configured to use the sensor's output value to detect both states of the pump: (1) engagement state between the DP and RP of the pump device, and (2) correctness of the engagement orientation. The controller may be configured to output (audibly and/or visually) a corresponding indication to the patient, for example "engaged", "disengaged", "correct orientation", "incorrect orientation", etc. In terms of engagement detection, DP **1320** includes only a simple, relatively inexpensive, metal plate, which makes DP **1320** more readily disposable after use.

Fig. 14 depicts a magnet-sensor combination **1400** according to an example embodiment. Magnet-sensor combination **1400** includes a flat, rounded (ring like), magnet **1410,** and a magnetic field sensor **1420.** As described herein in connection with other magnetic field sensors, magnetic field sensor **1420** lies in the X-Z plane and is positioned in the middle of (and circumferentially surrounded by) magnet **1410** and slightly sunk (indented) with respect to the outer surface (top surface in the drawing) of magnet **1410.** Positioning the magnetic field sensor slightly below the surface of the ring-like magnet **1410** and in the middle of it is beneficial in terms of the strength and direction of the magnetic fields in the space closely surrounding the magnetic field sensor. Namely, positioning a magnetic field sensor relative to a magnet in the way described herein and/or shown in the drawings (e.g., in Figs. 1, 2B, 3B, 4A-4B, 14, 15A-15B, 16A-16B) zeroes out the output signal of the magnetic field sensor when the RP and the DP of a pump device are disengaged, and improves the sensitivity of the magnetic field sensor to the magnetic disruption that the AMP causes in the magnetic field(s) when the RP and the DP are engaged.

Magnet **1410** may be a dipole magnet resembling, for example, dipole magnet **250** of Fig. 2B, or a multipole magnet resembling, for example, 4-pole magnet **410** of Fig. 4A. In some embodiments, a magnet may have more than four magnetic poles. In general, a magnet may include a number ***'n'*** (***n***=1, 2, 3,...) of pairs of conjugated magnetic poles (N/S), for example three (***n***=3) pairs of conjugated magnetic poles (i.e., three 'north' poles and three conjugated 'south' poles). A magnet having '***n***' pairs of conjugated magnetic poles may be magnetized diametrically, axially, or both diametrically and axially. Magnet-sensor combination **1400,** or a magnet-sensor combination similar to magnet-sensor combination **1400,** may be included in (e.g., be part of) a RP of a pump device, for example it may be part of RP **1310** of Figs. 13A-13B.

Magnet-sensor combination **1400** also includes an electrical terminal **1430** for electrically powering magnetic field sensor **1420** by a power source that may be located in the RP of the pump device, and for transferring the output signal of magnetic field sensor **1420** to a controller of the related RP, for example to the controller that is located in main body **1330** of RP **1310.** (The power source and the controller are not shown in Fig. 14.)

Common to all magnets, sensor's sensing plane and AMPs in all the drawings is that they are all in the X-Z plane (or parallel to the X-Z plane), with the Y-axis being normal (orthogonal) to their planes. Another feature that is common to all magnets, magnetic field sensors and AMPs is that the asymmetry line of the AMP (for example asymmetry line **780** of AMP **700,** asymmetry line **880** of AMP **800)** coincides with (or is parallel to) the Z-axis. Orienting the magnet, sensor's sensing plane and AMP in the way shown in the drawings and described herein facilitates optimization of the magnetic disruption by the AMP, hence the sensing and detection of the engagement state (including engagement orientation) by the controller of the pump device.

Fig. 15A and Fig. 15B respectively depict a partial length cross-sectional view **1500** and a partial width cross-sectional view **1502** of a DP and a RP of a pump device that are engaged, according to an example embodiment. Referring to Fig. 15A, the DP includes two medicament reservoirs **(1510, 1520)** that may be structurally similar to reservoirs **1360** and **1370** of Fig. 13A. Medicament reservoir **1510** is accessible (operable) via luer port **1530,** and medicament reservoir **1520** is accessible (operable) via luer port **1540.** Luer ports **1530** and **1540** are structurally similar to luer ports **1020** and **1030** of Fig. 10C.

The RP includes a magnet-sensor combination similar to magnet-sensor combination **1400** of Fig. 14, and the DP includes an asymmetrical metal plate (AMP). The magnet-sensor combination includes a ring like (ring shaped) magnet **1550** similar to magnet **1410** of Fig. 14, and a magnetic field sensor **1560** similar to magnetic field sensor **1420** of Fig. 14. The AMP is shown at **1570.** Also shown in Figs. 15A-15B is an electrical terminal **1580** for electrically powering magnetic field sensor **1560** by a power source (that is located in the RP of the pump device), and for transferring the output signal of magnetic field sensor **1560** to a controller for processing. (The power source and the controller are not shown in Figs. 15A-15B.)

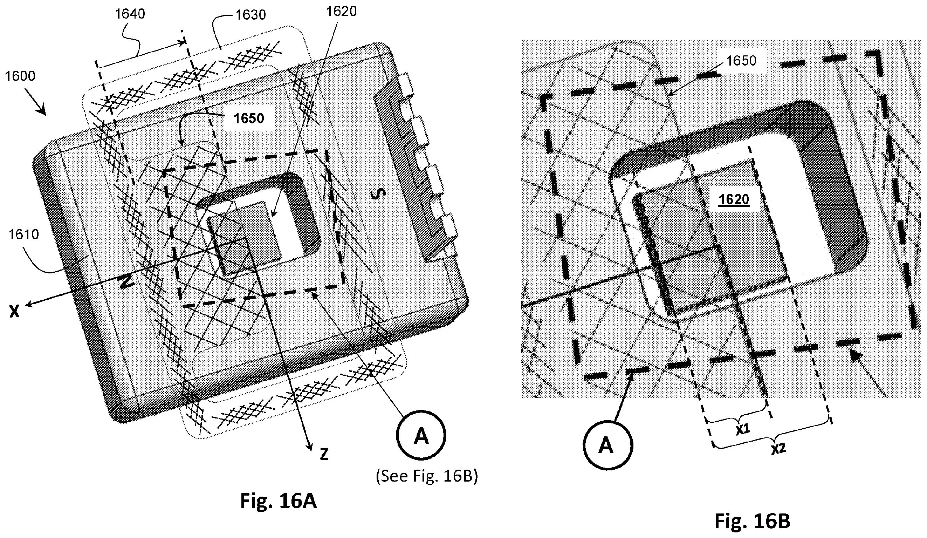

Figs. 16A-16B depict an example magnet-sensor-metal plate (MMP) setup according to an example embodiment. Figs. 16A-16B illustrate an overlapping relationship, ***P***[%], between a sensing area of a magnetic field sensor and an asymmetrical metal plate (AMP) according to an example embodiment. (Fig. 16B depicts an enlargement of detail A of Fig, 16A.)

A magnet-sensor combination **1600** includes a magnet **1610** and magnetic field sensor **1620.** Magnet-sensor combination **1600** and AMP **1630** are designed such that when the related DP and RP of a pump device are engaged, tab **1650** partially overlaps magnetic field sensor **1620.** (The greater the inward protrusion **1640** of tab **1650;** i.e., the greater the value of ***X1*** in Fig. 16B, the greater the overlap between tab **1650** and the sensing area of magnetic field sensor **1620.)** The overlap percentage **'*P*'** may be calculated as ***P**=*(***X1***/***X2***)x100. (Depending on the used AMP, ***Xi*** may denote length or area.)

If the overlap percentage is zero (if ***X1***=0), the disruption that tab **1650** causes to the magnetic field(s) produced by magnet **1610** may not be detectable by magnetic field sensor **1620,** or the difference between the sensor's output signal in the 'disengagement' state and the sensor's output signal in the 'engagement' state may be too small for a controller to reliably distinguish between the two states. In addition, if ***X1***=0 the magnetic attraction force that is induced between tab **1650** and magnet **1610** may be weak for proper attachment of the DP to the RP of the pump device. On the other hand, a configuration in which ***X1**=**X2*** also poses an issue that is related to the disruption (magnetic deflection) that the AMP induces in the magnetic field(s). That is, if the overlap percentage is 100%, or near 100%, the disruption that tab **1650** induces in the magnetic field(s) produced by magnet **1610** may be so great that magnetic field sensor **1620** may enter saturation state even before the DP and the RP of the pump device are engaged. As a result of this, the output signal (saturation value) of magnetic field sensor **1620** may erroneously be interpreted by the controller as indicating engagement of the DP and RP even before engagement occurs. Briefly, a sensor's saturation is a state in which the actual signal that needs to be measured is unmeasurable because it exceeds the output dynamic range of the sensor. Therefore, the saturation value of the sensor's output becomes a limiting value of the sensor's dynamic output range. (The lower the sensor's saturation value, the smaller the sensor's dynamic output range.) In addition, if ***X1**=**X2**,* the magnetic attraction force that is induced between tab **1650** and magnet **1610** may be too strong for a user (e.g., PD patient) to disengage the DP from the RP of the pump device.

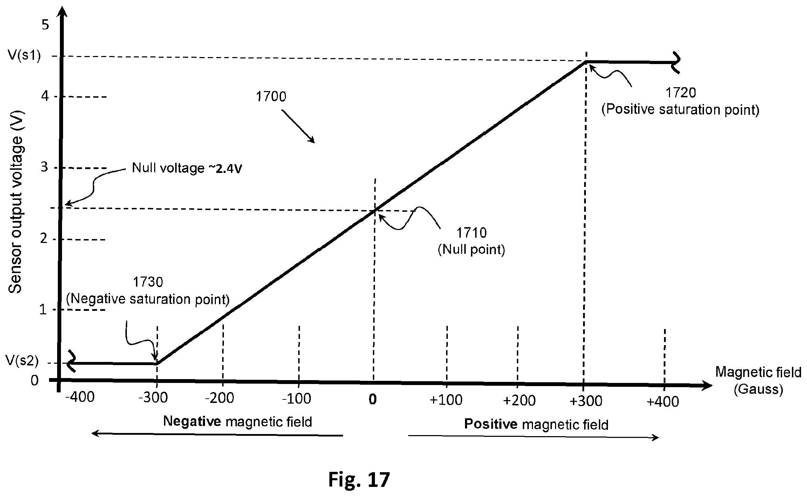

Turning to Figs. 16A-16B, entering the sensor's saturation state may cause a considerable error between the actual (true) distance (spacing) between AMT **1630** and sensor **1620** and the distance (spacing) as estimated by the controller from the sensor's saturation output value. (Example saturation points of a magnetic field sensor are shown in Fig. 17.)

Given the two constraints (i.e., detectable magnetic disruption and preferable magnetic attraction force), the value of ***X1*** should preferably be greater than zero to obtain a strong enough magnetic attraction force and a detectable magnetic disruption, and smaller than ***X2*** to prevent a too strong magnetic attraction force and/or entering saturation (i.e., ***X1*** has to meet the condition **0*<X1<X2*).** A traded off overlapping value may be, for example, 50% (i.e., ***X1**=**X2***/2)*.* The two constraints mentioned with regard to the overlap percentage, **P,** apply also to AMP **500,** AMP **600,** AMP **700,** and AMP **800,** as well as to AMPs of other designs.

Fig. 17 shows an example output response of a linear Hall effect sensor. A Hall effect sensor can operate as an analog transducer, directly returning a voltage that is proportional to the magnetic field that is sensed by the sensor. The Hall effect sensor can be sensitive to both positive fields and negative fields. A linear Hall effect sensor can provide a linear response similar to the response shown in graph **1700** by applying a fixed offset (null point **1710** corresponding to null voltage ^{~}2.4V) to the output of the sensor when no magnetic field is sensed by the Hall effect sensor. When a positive magnetic field increases above zero Gauss ("G"), the sensor's output voltage increases linearly above null voltage **1710** until it reaches positive saturation point **1720** (i.e., positive saturation value ***V(s1)*** at +300[G]) Similarly, when a negative magnetic field increases in the opposite direction (i.e., below zero Gauss), the sensor's output voltage increases linearly with respect to null voltage **1710** until it reaches negative saturation point **1730** (i.e., negative saturation value ***V(s2)*** at -300[G]. So, in the example of Fig. 17 the sensor's output dynamic range (voltage) spans between ***V(*s*1)*** and ***V(s2)**,* and the sensor can reliably measure a magnetic field strength that spans between -300[G] and +300[G].

Turning back to Fig. 13A, it shows DP **1320** of pump device **1300** before it is attached to RP **1310** in a first orientation, where driving nut **1362** is to engage driving gear **1332** and driving nut **1372** is to engage driving gear **1334.** However, thanks to the structural symmetry of DP **1320** with respect to middle leg portion **1340** of RP **1310,** the DP and RP can also be attached in a second orientation where one part (e.g, DP **1320**) is rotated 180 degrees with respect to the first orientation (i.e., it is rotated about the Y-axis), such that driving nut **1362** would engage driving gear **1334**, and nut **1372** would engage driving gear **1332.**

This feature can enhance the usability of the pump device **1300** because a patient does not need to be hassled by the orientation between RP **1310** and DP **1320** when attaching the two parts. In other embodiments, though, RP **1310** and DP **1320** may have to be engaged in a particular orientation in order to make the pump device operable. According to the present invention, ensuring that the engagement orientation of the DP is the operational orientation may be done by using the asymmetry feature of the asymmetrical metal plate (AMP), as described herein.

As described herein and shown in the drawings, asymmetrical metal plates (AMPs) are asymmetric with respect to the Z-axis (see, for example, AMPs **500**, **600**, **700**, **800** and **1630).** Therefore, rotating the DP of a pump device 180 degrees in the X-Z plane (rotating it about the Y-axis) relative to the RP also rotates the AMP 180 degrees in the X-Z plane (about the Y-axis) relative to the magnetic field sensor. Thanks to the asymmetry of the AMP with respect to the Z-axis, the magnetic disruption that the AMP induces in the magnetic field(s) at the sensor in the first engagement orientation differs from the magnetic disruption induced by the AMP at the sensor in the second engagement orientation. Consequently, the output signal of the Hall effect sensor for the first engagement orientation differs from the output signal of the Hall effect sensor for the second engagement orientation. Therefore, the output signal of the magnetic field sensor with respect to the null voltage (null point **1710** in Fig. 17) may be used to distinguish between the two engagement orientations of the DP, for example in the way described in connection with Figs. 18A-18B

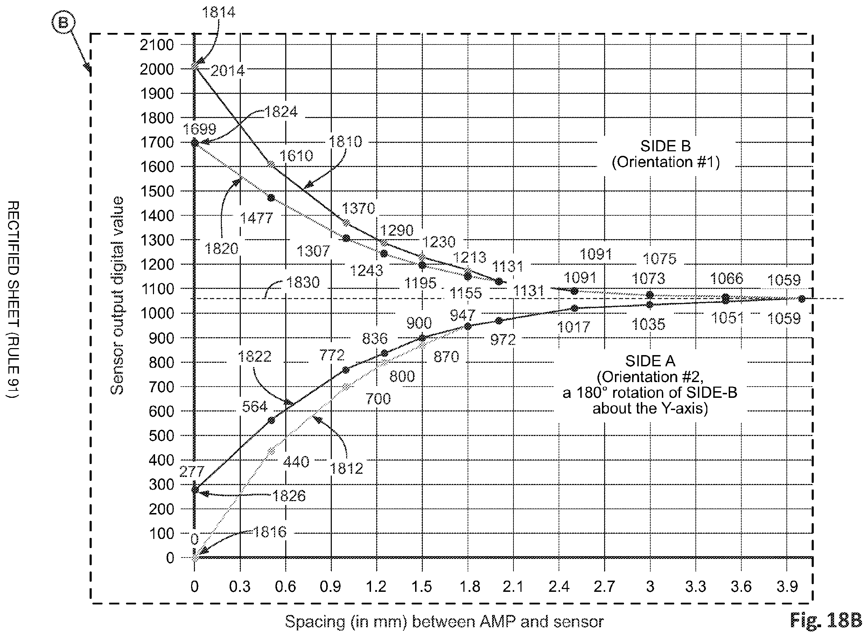

Figs. 18A-18B are comparative figures showing 'saturation' curves **1810** and **1812** vis-a-vis non-saturation curves **1820** and **1822,** according to an example embodiment. (Fig. 18B shows an enlargement of item **B** in Fig. 18A.) Curves **1810** and **1812** are associated with an AMP designed such that the AMP causes a magnetic field sensor (e.g., sensor **1420,** Fig. 14) to enter saturation state. In the example shown in Figs. 18A-18B, the sensor's output saturation values are *'2014'* for magnetic fields in a first direction, and zero (*'0'*) for magnetic fields in a second direction that is opposite the first direction. Accordingly, the operable dynamic output range [*0÷2014*] of the sensor is, in this example, the largest possible range. (Saturation values of a magnetic field sensor generally depend on the type of the sensor and on the design of the related electrical circuit, for example the operating voltage(s), etc.)

Curve **1810** represents the sensor's output value (i.e., digital value) as a function of the distance, or gap/spacing, between the AMP and the sensor when the AMP is oriented in a first orientation relative to the sensor. (The first orientation is referred to herein as orientation 'SIDE-B'). Curve **1812** represents the sensor's output value as a function of the distance, or gap/spacing, between the AMP and the sensor when the AMP is oriented in a second orientation ('SIDE-A') relative to the sensor. (The second orientation is referred to herein as orientation 'SIDE-A'). The second orientation ('SIDE-A') of the AMP is obtained by rotating the AMP 180 degrees in the X-Z plane, about the Y-axis. (See plane X-Y and the Y-axis in, for example, Fig. 10C.) For ease of understanding, the relative spatial positioning of the AMP, magnet, magnetic field sensor, DP and RP of the pump device is shown using the same X-Y-Z coordinate system, which is shown, for example, in Figs. 2B, 4B, 5B, 6B, 7B, 10C, 13A, 15A-15B and 16A.) Curves **1820** and **1822** are respectively similar to curves **1810** and **1812,** except for a difference which is that curves **1820** and **1822** are associated with an AMP design that avoids sensor saturation in order to leave a useful (e.g., safety) output margin, with respect to the saturation value(s) of the sensor, when the DP and RP are engaged. (Curves **1810, 1812, 1820** and **1822** were obtained by using a simulation method.)

### Orientation 'SIDE-B': saturation versus non-saturation

Referring to curve **1810,** when the AMP is distanced away from the magnetic field sensor to a distance greater than 3.6mm (in this example), the sensor's output value (a digital code/value, ***Sa***) is its null value ***Sₙᵤₗₗ*** (***Sₙᵤₗₗ** = ~1,060),* because at a distance greater than 3.6mm the AMP does not disrupt the magnetic field(s) at the sensor, so the net magnetic field sensed by the sensor is zero, or near zero, which corresponds to the sensor's null value *~1,060*. (Null line **1830** corresponds to null value ***Sₙᵤₗₗ** = ~1,060* of the sensor.) However, as the AMP (in orientation 'SIDE-B') is brought closer to the sensor, the magnetic disruption caused by the AMP gradually increases, which results in a corresponding increase in the sensor's output value, until at zero distance the sensor outputs the upper saturation value *'2014'* (saturation point **1814** in Figs. 18A-18B).

Referring to curve **1820,** it illustrates a similar dependency between spacing (between the AMP and the sensor) and the sensor's output value as curve **1810.** Namely, as the AMP (also in orientation 'SIDE-B') is continually brought closer to the sensor, the magnetic disruption caused by the AMP gradually increases, which results in a corresponding increase in the sensor's output value, until at zero distance the sensor outputs the non-saturation value *'1699'* (point **1824** in Fig. 18B). So, a sensor's output value *'1699'* at the zero spacing leaves a 'safety' margin of *'315*' (i.e., *2014-1699=315*).

The safety margin is useful, for example, in detecting a malfunctioning sensor (or another component of the electrical circuit) by a controller of the RP of the pump device when the DP and the RP of the pump device are engaged. For example, if the DP and RP of the pump device are engaged but the sensor outputs the saturation value, the controller may determine that the sensor, or some other electrical component, does not function properly, and, accordingly, may respond by outputting (audibly and/or textually) a warning message for the patient and simultaneously stop delivering medicament from the pump device to the patient. (This feature is useful for all malfunctions that, upon occurrence, cause the magnetic field sensor to enter the saturation state.) So, in terms of detecting malfunctions, using a 'non-saturation' curve similar to curve **1820** is beneficial comparing to using a saturation curve similar to curve **1810.**

### Orientation 'SIDE-A': saturation versus non-saturation

Referring to curve **1812,** when the AMP is distanced away from the magnetic field sensor to a distance greater than 3.6mm (in this example), the sensor's output value is its null value/point (*~1,060*) because at a distance greater than 3.6mm the AMP does not disrupt the magnetic field(s) at the sensor, so the net magnetic field sensed by the sensor is zero, or near zero, which corresponds to the sensor's output value *~1,060.* However, as the AMP (this case in the 'SIDE-A' orientation) is brought closer to the sensor, the magnetic disruption caused by the AMP gradually increases, which results in a corresponding increase in the sensor's output value, until at zero distance the sensor outputs the low saturation value '*0*' (point **1816** in Figs. 18A-18B).

Referring to curve **1822,** it illustrates a similar dependency between spacing (between the AMP and the sensor) and the sensor's output value as curve **1812.** Namely, as the AMP (in orientation 'SIDE-A') is continually brought closer to the sensor, the magnetic disruption caused by the AMP gradually increases, which results in a corresponding increase in the sensor's output value, until at zero distance the sensor outputs the non-saturation value *'277'* (point **1826** in Fig. 18B). So, a sensor's output value '*277*' at the zero spacing leaves a safety margin of '*277*' (i.e., *277-0=277*). Similarly to curve **1820,** curve **1822** also provides a safety margin that is useful, for example, in detecting a malfunctioning sensor (or another component of the electrical circuit) by a controller of the RP of the pump device when the DP and the RP of the pump device are engaged.

### Magnitude (M) of the sensor's output at zero spacing: saturation vs. non-saturation

A magnitude, '***M***', of the sensor's output for each distance between the AMP and the sensor may be calculated as ***M**=*|***Sa** - **Sₙᵤₗₗ***|*,* where *'**Sa**'* is the sensor's actual (e.g., measured) output value, and *'**Sₙᵤₗₗ**'* is the sensor's null value. Regarding 'saturation' curves **1810** and **1812,** the sensor's output magnitude ***M*** for zero distance in the first orientation (curve **1810,** 'SIDE-B') of the AMP is *954* (***M**=*|*2014-1060*|*=954*)*.* In the second orientation (curve **1812,** 'SIDE-A') of the AMP, the sensor's output magnitude ***M*** for zero distance is *1060* (***M**=*|0-1060|*=1060*). Regarding 'non-saturation' curves **1820** and **1822,** in the first orientation (curve **1820,** 'SIDE-B') of the AMP the sensor's output value ***M*** for zero distance is 639 (***M***=|*1699-1060*|=*639*), and in the second orientation (curve **1822,** 'SIDE-A') of the AMP the sensor's output value ***M*** for zero distance is *783* (***M**=* |*277-1060*|=*783*).

The sensor's output magnitude, **'*M*',** or span, for non-saturation curves is smaller comparing to the sensor's output magnitude, **'*M*',** or span, for saturation curves. However, as described herein, non-saturation curves provide a useful safety margin. The safety margin may be designed as a tradeoff between the sensor's output magnitude, '***M**'*, and the ability of the pump's controller to detect a faulty magnetic field sensor and/or other circuit components. In other words, it would be beneficial to increase the value of ***M*** without sacrificing the controller's ability to detect a faulty magnetic field sensor, and/or other circuit components, by using the sensor's saturation value(s).

### Null point and distinguishing between orientation 'SIDE-B' and orientation 'SIDE-A'

As described in connection with Fig. 17, the magnetic field sensor has a null voltage **1710** that divides the sensor's output dynamic range into 'positive' and 'negative' segments that respectively correspond to positive and negative magnetic field directions. (The null value of a bipolar magnetic field sensor is the signal (analog or digital) that the sensor outputs when it does not sense any magnetic field.) In the example of Figs. 18A-18B, the null value, ***Sₙᵤₗₗ**,* is digital value ~1060, and it is used to distinguish between the two orientations ('SIDE-B', 'SIDE-A') of the AMP, because all sensor output values related to the first orientation ('SIDE-B') are greater than null value ~1060 (all output values are above null line **1830),** whereas all sensor output values related to the second orientation ('SID-A') are smaller than null value ~1060 (all sensor output values in this case are below null line **1830).**

Curves **1812** is roughly a mirror image of curve **1810** with respect to null line **1830.** Similarly, curve **1822** is roughly a mirror image of curve **1820** with respect to null line **1830.** 'Mirror' curves such as curves **1812** and **1822** are the result of the AMP being asymmetrical, with the asymmetry line coinciding with the Z-axis. Imparting asymmetry to the AMP and positioning (subtending) the AMP against the sensor (and against the magnet that surrounds the sensor) such that the AMP's asymmetry line is perpendicular to the X-axis enables to reverse the direction of the magnetic field that is sensed by the magnetic field sensor due to the magnetic disruption caused by the AMP. That is, if the AMP is positioned against (if it subtends) the sensor in orientation 'SIDE-B', the magnetic field that the sensor senses due to the magnetic disruption of the AMP is in a first direction, which results, in this example, in curve **1820.** However, if the AMP is rotated 180 degrees with respect to the Y-axis (i.e., positioned in orientation 'SIDE-A'), the direction of the magnetic field that the sensor senses due to the magnetic disruption of the AMP at this orientation is reversed, which results, in this example, in curve **1822.**

### Example

Applying curve **1810** (Fig. 18A-18B) to Figs. 15A-15B (which show example magnet **1550,** sensor **1560** and AMP **1570),** when the DP is not engaged with the RP and the distance (spacing) between AMP **1570** and sensor **1560** is, in this example, greater than about 3.6mm, the magnetic field(s) produced by magnet **1550** is(are) undisrupted (or the magnetic disruption is undetectable) and, therefore, the net magnetic field that sensor **1560** senses is zero, or near zero. Therefore, the output signal of sensor **1560** is the null value *~1060* (see null line **1830).** When the DP (with AMP **1570)** is brought closer to the RP (with its sensor **1560),** namely when the spacing between them is less than about 3.6mm during engagement, the magnetic disruption that AMP **1570** induces at sensor **1560** increases as AMP **1570** is moved closer to sensor **1560.** As a result of the increase in the magnetic disruption at the sensor, the output value of sensor **1560** also increases. (The closer AMP **1570** is to sensor **1560,** the greater is the sensor's output value due to the increasing magnetic disruption.)

Assuming that the saturation value of magnetic field sensor **1560** is *2014* (per sensor response curve **1810),** AMP **1570** may be designed such that curve **1810** reaches the sensor's saturation value *2014* when the spacing between AMP **1570** and sensor **1560** is zero, or near zero. On the one hand, designing AMP **1570** to cause the sensor's output value to be its saturation value (in this example *2014*) for zero spacing means maximizing the resolution of the spacing measurement, hence spacing measurement precision. On the other hand, if the DP and the RP are engaged (if the spacing between them is zero) but the sensor, or associated electronics, is faulty or it malfunctions, the faulty condition cannot be detected by using the sensor's output signal because the saturation output value of the sensor can be used to detect (i.e., be associated with) either a zero spacing between an AMP and a sensor (i.e., detect engagement state), or a circuit problem, but not both. To enable using the sensor's output signal to detect both zero (or near zero) AMP-sensor spacing and a faulty sensor, electronics or software, AMP **1570** can be designed in a way that the sensor's output value would be 'slightly' lower (e.g., *1699,* per sensor response curve **1820)** than the sensor's saturation output value *2014* when the spacing between AMP **1570** and sensor **1560** is zero, or near zero. Curve **1820** solves this problem because, on the one hand, the sensor's output value *1699* indicates when the AMP-sensor distance is zero (or near zero), and, on the other hand, in case the sensor and/or any other system component, is faulty, the sensor's output would 'jump' to its saturation value. So, when the DP and RP of the pump device are engaged, the two sensor output values *1699* and *277* enable (e.g., the controller in the RP) to distinguish the engagement state from a malfunctioning condition. AMP **1570** (for example) can be designed accordingly to impart this kind of 'under saturation' output-distance response curve to sensor **1560.**

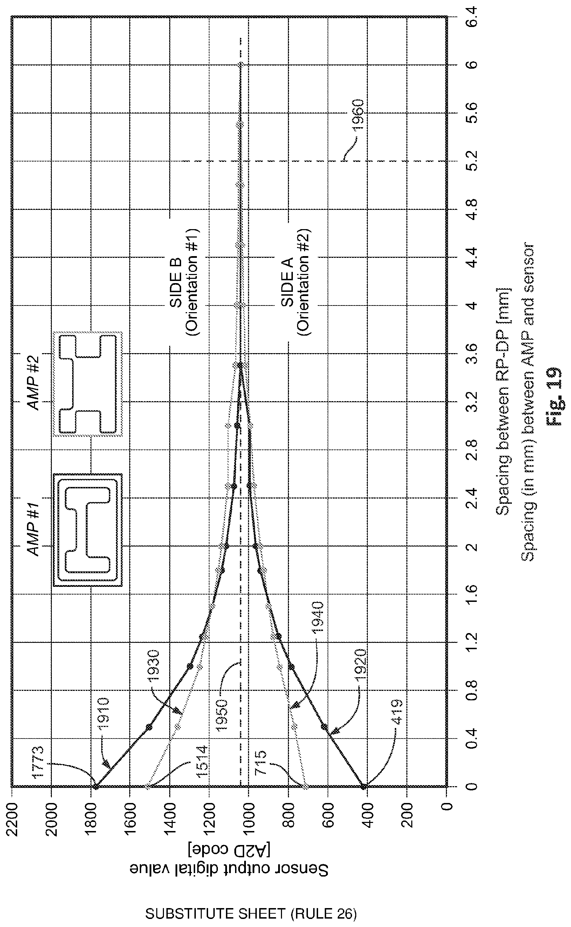

Fig. 19 shows example comparative sensor output response curves **1910** and **1920** for two different AMP designs in accordance with an example embodiment. The two AMPs compared in Fig. 19 are AMP **800** of Fig. 8, which is referred to in Fig. 19 as *'AMP #1',* and AMP **500** of Figs. 5A-5C, which is referred to in Fig. 19 as *AMP #2.* Curves **1910** and **1920** embody simulation results for *AMP #1.* Curves **1930** and **1940** embody simulation results for *AMP #2.*

Comparing curves **1910** and **1930** shows that the design of *AMT #1* is beneficial over the design of *AMP #2* because the sensor's output range (magnitude ***M***) resulting from design *AMP #1* is significantly larger in span at the zero spacing (e.g., *1773-1040=733*) than the sensor's output range resulting from design *AMP #2* at the same spacing (i.e., *1514-1040=474).* Comparing the respective 'mirror' curves **1920** and **1940** shows similar behavior. (Sensor's output value *1040* is the sensor's null value corresponding to null line **1950.)** In addition, the design of *AMP #1* provides a greater magnetic attraction force comparing to the design of *AMP #2,* and this magnetic property applies both to the 2-pole magnet configuration and to the 4-pole magnet configuration, as the specific example comparative information below demonstrates:

### Typical magnetic attraction force for a 2-pole magnet configuration:

*1. AMP #1:* 7.0[N].
*2. AMP #2:* 4.5[N].

### Typical magnetic attraction force for a 4-pole magnet configuration:

*1. AMP #1:* 11.0[N].
*2. AMP #2:* 6.0[N].

Example factors to be considered when designing an AMP:
1. The design of the AMP (e.g., AMP **500,** AMP **600,** AMP **700,** AMP **800,** etc.) dictates the 'elevation' of the sensor's output response curve (in orientation 'SIDE-B') with respect to the sensor's null value/line, or the 'lowering' of the curve (in orientation 'SIDE-A') with respect to the sensor's null value/line. The design of the AMP also dictates the curvature of the sensor's output response curve. Fig. 19 shows an example effect of this factor.
   Fig. 19 shows two sensor output response curves of *AMP #1* (e.g., curve **1910** for the 'SIDE-B' orientation of AMP #1, and curve**1920** for the 'SIDE-A' orientation of *AMP #1*) vis-à-vis two sensor output response curves of *AMP #2* (e.g., curve **1930** for the 'SIDE-B' orientation of AMP #2, and curve**1940** for the 'SIDE-A' orientation of AMP #2).
   The different design of these two AMPs (*AMP #1* and *AMP #2*) causes the sensor to respond differently for the same distance between the AMPs and the sensor. For example (referencing the 'SIDE-B' orientation in Fig. 19), *AMP #1* causes the sensor to output the value *1773* (on curve **1910)** for zero 'AMP-sensor' distance, and *AMP #2* causes the sensor to output a different value (e.g., value *1514* on curve **1930)** for the same zero 'AMP-sensor' distance/spacing/gap. The 'SIDE-A' orientation of *AMP #1* and *AMP #2* results in a similar difference between the sensor's output values (e.g., value *419* on curve **1920** associated with AMP #1, versus value *715* on curve **1940** associated with *AMP #2.* Similar differences in the sensor's output values occur from the zero AMP-sensor distance up to an *'Engagement Threshold Distance ("ETD"), **Dₜₕ,*** (As illustrated in, for example, Fig. 19, the sensor's output is the null value *~1040* (null line **1950)** for all AMP-sensor distances that are greater than ***Dₜₕ*** (in this example ***Dₜₕ***≅5.2mm). ***Dₜₕ*** is shown in Fig. 19 at **1960.)**
2. The type of the sensor is a factor determining the magnitude of the sensor's output signal for a given AMP design and AMP-sensor distance.
3. The preferable magnetic attraction force between the AMP and the magnet. The greater the preferable magnetic attraction force, the greater the area of the AMP that is required. Increasing the size of the AMP may increase the AMP's effect on the sensor's output response up to entering saturation.
4. The actual 'zero' distance between the AMP and the sensor after assembling of DP and RP of the pump device. The distance between the AMP and the sensor may be subjected to constraints imposed by the design of the DP and RP of the pump device. This practically means that in one design the engagement distance between the AMP and the sensor may be, for example, 0.00mm, while in another design it may be, for example, 0.05mm, 1.3mm, 2.0mm, etc.
5. The electronics and operational voltage(s). For example, changes in the supply voltage may result in a change in the sensor's maximal output span.

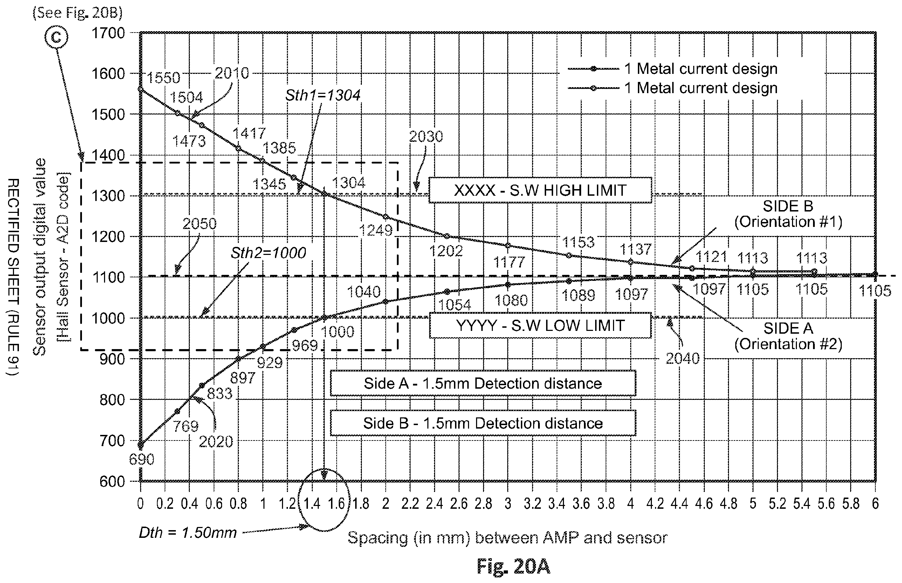

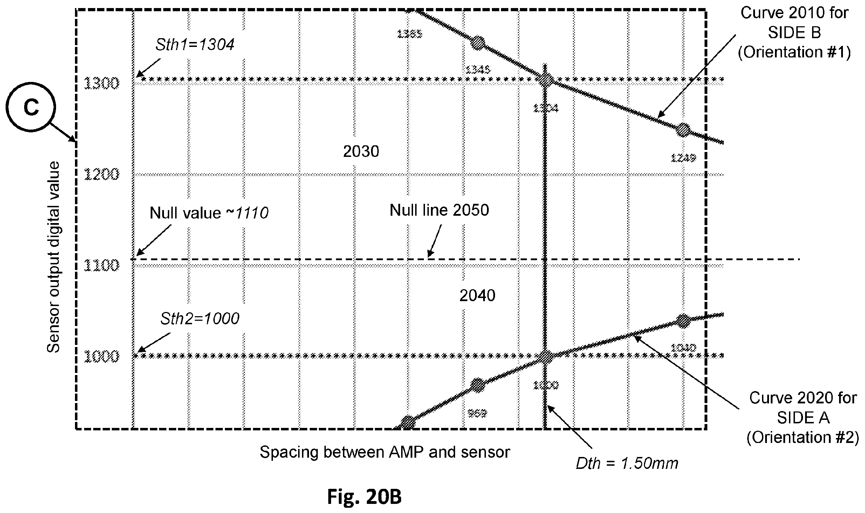

Figs. 20A-20B illustrate an engagement threshold distance/spacing calibration according to an example embodiment. *Engagement Threshold Distance (ETD), Dₜₕ,* is a threshold distance or spacing between an AMP and a magnetic field sensor that a controller (for example) uses to distinguish between engagement state and disengagement state between a DP and RP of a pump device. If the actual distance, ***Dₐ,*** as detected by the controller between the AMP and the sensor, is greater than the *ETD* (i.e., if ***Dₐ**>**Dₜₕ***), the controller determines that the DP of the pump device is not engaged with the RP of the pump device. However, if the actual distance ***Dₐ*** between the AMP and the sensor is equal to, or less than, the *ETD* (i.e., if ***Dₐ*** ≤ ***Dₜₕ***), the controller determines that the DP and the RP of the pump device are engaged.

During normal operation of the pump device the controller of the pump device determines the distance, ***Da**,* between the AMP and the magnetic field sensor from the sensor's output value. Since the relationship between the sensor's output value and the related distance/spacing between the AMP and the sensor vary from one pump design to another, a calibration process has to be performed in order to associate the sensor's threshold output value ***(Sth)*** with a threshold distance **(*Dth*)** that are specific to the pump device that is the subject of the calibration. Briefly, calibration is performed by engaging the DP with the RP and reading the sensor's output value *(Sth)* corresponding to the engagement distance. Assuming that the engagement orientation is 'SIDE B', any sensor's output value that is equal to or greater than the value of ***Sth*** during normal operation of the pump device would indicate that the DP and RP of the pump device are engaged. The calibration described herein applies to any design of AMP, magnetic field sensor, magnet, DP and RP of a pump device.

Curve **2010** represents an output value of a sensor (e.g., sensor **1560** of Figs 15A-15B) as a function of the distance, or gap/spacing, between an AMP (e.g., AMP **1570** of Figs 15A-15B) and the sensor when the AMP (hence the DP) is oriented in a first engagement orientation ('SIDE-B') relative to the sensor (hence to the RP). Curve **2020** represents the sensor's output value as a function of the distance, or gap/spacing, between the AMP and the sensor when the AMP is oriented in a second engagement orientation ('SIDE-A') relative to the sensor. (Curves **2010** and **2020** were obtained by using a simulation method.)

As described herein in connection with the two engagement orientations ('SIDE-A' and 'SIDE-B') of the AMP, the second engagement orientation ('SIDE-A') of the AMP is obtained by rotating the AMP 180 degrees in the X-Z plane, about the Y-axis. (The X-Y-Z coordinate system used, for example, in Figs. 2B, 4B, 5B, 6B, 7B, 10C, 13A, 14 and 15A-15B is common (applicable) to every AMP, sensor, magnet, DP and RP of the pump device that is shown in the drawings and described herein.)

At a first calibration step, a DP including an AMP is engaged in a first engagement orientation (e.g., orientation 'SIDE-B') with a RP including a magnet, a magnetic field sensor and a controller, and the magnetic field sensor outputs a first value, ***Sₜₕ₁**,* that corresponds to the *Engagement Threshold Distance* (*ETD*), ***Dₜₕ.*** In this example the engagement threshold distance is 1.5mm (i.e., ***Dₜₕ***=1.5mm), and the sensor's output value (engagement value, ***Sₜₕ₁***) corresponding to the aforesaid engagement distance is *1304* (i.e., ***Sₜₕ₁***=*1304*). All sensor output values, ***Sa**,* for the 'SIDE-B' orientation are above null line **2050** (i.e., all sensor's output values are greater than the null value ~*1110*).

Any sensor's output value, ***Sa**,* that is equal to or greater than *1304* **(*Sth1*)** indicates that the engagement (in orientation 'SIDE-B') is maintained. If the sensor's output value, ***Sa**,* gets smaller than *1304,* i.e., if *1110<**Sa**<1304* (*'1110'* is the sensor's null value, ***Sₙᵤₗₗ***), this indicates (e.g., to the controller) that the distance between the AMP and the sensor is greater than ***Dth*** (i.e., ***Da**>**Dth***). This means that the DP and the RP of the pump device have been disengaged. So, the sensor's output value ***Sₜₕ₁**=1304* is a first calibration value that the controller of the pump device uses to distinguish between engagement state and disengagement state in the first orientation (the 'SIDE-B' orientation) of the DP relative to the RP.

At a second calibration step, the DP is engaged with the RP in the second orientation (e.g., the orientation corresponding to 'SIDE-A'), and the magnetic field sensor outputs a second value, ***Sth2,*** that reflects the engagement state in the second orientation ('SIDE-A' orientation). In this example the engagement distance is also 1.5mm (i.e., ***Dₜₕ***=1.5mm), and the sensor's output value (engagement value ***Sₜₕ₂*)** is *1000* (i.e., ***Sₜₕ₂**=1000*). (The engagement distance for both orientations is, in this example, 1.5mm. However, depending on the design of the pump device, the engagement distance may change from one pump device to another, or from one engagement orientation to another.) All sensor output values, ***Sa**,* for the 'SIDE-A' orientation are below null line **2050** (i.e., all sensor's output values are smaller than the null value *~1110*).

Any sensor's output value, ***Sa***, that is equal to or smaller than *1000* indicates that the engagement (in orientation 'SIDE-A') is maintained. If the sensor's output value, ***Sa**,* is greater than *1000,* i.e., if *1000<**Sa**<1110* ('*1110*' is the null value, ***Sₙᵤₗₗ***), this is an indication that the DP and RP of the pump device have been disengaged. So, the sensor's output value ***Sth2**=1000* is another calibration value that the controller of the pump device uses to distinguish between engagement state and disengagement state in the second engagement orientation (the 'SIDE-A' orientation) of the DP relative to the RP.

The controller of the pump device can, thus, determine two things from a single sensor's output value: (1) engagement state ("engaged", "disengaged"), and (2) engagement orientation of the DP (engagement orientation 'SIDE-B' or 'SIDE-A'). For example, a controller of the pump device may determine the engagement orientation (e.g., 'SIDE-B' or 'SIDE-A') of the DP relative to the RP by comparing the sensor's output value to the sensor's null value, ***Sₙᵤₗₗ**,* and determining whether the sensor's output value is greater, equal to or smaller than the sensor's null value ***Sₙᵤₗₗ**.* For example, if a sensor's output value, ***Sa**,* is greater than the sensor's null value (i.e., if ***Sa**>**Sₙᵤₗₗ***)*,* the engagement orientation is 'SIDE-B', and if the sensor's output value is smaller than the sensor's null value (i.e., if ***Sa**<**Sₙᵤₗₗ***)*,* the engagement orientation is 'SIDE-A'.

Regarding determination of the engagement state ('engaged' or 'disengaged'), if the sensor's output value, ***Sa**,* is equal to or greater than the first threshold value (i.e., if ***Sa***≥***Sₜₕ₁***)*,* the controller determines that the DP is engaged with the RP in engagement orientation 'SIDE-B'. Similarly, if the sensor's output value, ***Sa**,* is less than the second threshold value (i.e., if ***Sa**<**Sₜₕ₂***), the controller determines that the DP is engaged with the RP in engagement orientation 'SIDE-A'.

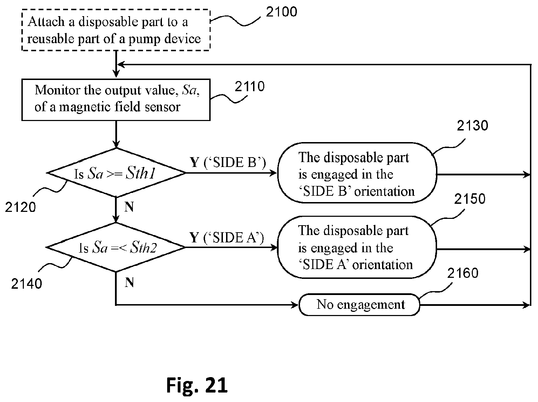

So, during the calibration process the controller monitors the sensor's output value for both engagement orientations ('SIDE-B' and 'SIDEA') of the DP, and terminates the calibration process after having identified two engagement threshold values, ***Sₜₕ₁*** and ***Sₜₕ₂**,* where each engagement threshold value corresponds to a specific engagement orientation. (Engagement threshold value ***Sₜₕ₁*** corresponds to engagement orientation 'SIDE-B', and engagement threshold value ***Sth2*** corresponds to engagement orientation 'SIDE-A'.) Then, the controller uses the two engagement threshold values **(*Sₜₕ₁, Sₜₕ₂*)** to detect the engagement orientation during normal operation of the pump device. The way the controller uses the two engagement threshold values is shown in Fig. 21, which is described below. The calibration process, which 'pairs' a DP with a specific RP, may be part of the manufacturing and/or assembly process of the pump device.

Fig. 21 shows a method of determining an engagement between a DP and a RP of a pump device according to an example embodiment. As described herein (for example in connection with Figs. 17, 18A-18B and 20A-20B), the null value, ***Sₙᵤₗₗ**,* enables distinguishing between engagement orientation SIDE-B and engagement orientation SIDE-A. The engagement determination method of Fig. 21 is based on the relationships between the two calibration threshold values **(*Sth1, Sth2*),** which are described in connection with Figs. 20A-20B, and the sensor's null value (***Sₙᵤₗₗ***)***,*** namely, on the relationship expression ***Sth2** < **Sₙᵤₗₗ** < **Sth1.***

At step 2100, a DP of the pump device (e.g., DP **1310** of Fig. 13A) is magnetically engaged with a RP of the pump device (e.g., RP **1320** of Fig. 13A) by magnetic attraction force that is induced between an AMP in the DP (e.g., AMP **1380** of Fig. 13A) and a magnet in the RP (e.g., the magnet at **1350** in Fig. 13A). At step 2110 a controller included in the RP of the pump device continually reads an output value, ***Sa**,* of a magnetic field sensor that is included in the RP of the pump (e.g., the sensor at **1350** in Fig. 13A).

At step 2120, the controller compares the value of ***Sa*** to the first engagement threshold value, ***Sth1**,* to check whether the DP and the RP are engaged in orientation 'SIDE-B'. If the value of ***Sa*** is equal to or greater than the value of ***Sth1*** (this condition is shown as "Y" at step 2120), the controller determines, at step 2130, that the DP is engaged with the RP in the 'SIDE-B' orientation, and continues to monitor, at step 2110, the value of ***Sa*** to check whether the engagement is maintained or disrupted. In case of disengagement, or disrupted engagement, if this occurs during delivery of medicament to the patient, the controller stops delivering the medicament and outputs (audibly and/or visually) a corresponding alarm to the pump user (e.g., a patient). However, if the value of ***Sa*** is smaller than the value of ***Sth1*** (this condition is shown as "N" at step 2120), the controller determines that the DP is not engaged with the RP in the 'SIDE-B' orientation, and proceeds to check potential engagement in the 'SIDE-A' orientation.

At step 2140, the controller compares the value of ***Sa*** to the second engagement threshold value, ***Sth2.*** If the value of ***Sa*** is equal to or smaller than the value of ***Sth2*** (this condition is shown as "Y" at step 2140), the controller determines, at step 2150, that the DP is engaged with the RP in the 'SIDE-A' orientation, and continues to monitor, at step 2110, the value of ***Sa*** to check whether the engagement is maintained or disrupted. In case of disengagement, or disrupted engagement, if this occurs during delivery of medicament to the patient, the controller stops delivering the medicament and outputs (audibly and/or visually) a corresponding alarm to the patient. However, if the value of ***Sa*** is greater than the value of ***Sth2*** (this condition is shown as "N" at step 2140), the controller determines, at step 2160, that the DP is not engaged with the RP in any orientation (i.e., neither in the 'SIDE-A' orientation, nor in the 'SIDE-B' orientation), and continues to monitor, at step 2110, the value of ***Sa*** to detect the engagement state ('engaged', 'engagement orientation', 'disengaged') of the pump device at any given time. The controller may check the value of ***Sa*** continuously, using a predetermined time interval that may change, for example, according to the operation mode of the pump device. For example, during normal delivery of medicament to the patient the controller may check the value of ***Sa*** frequently, for example once every ***t1*** seconds, and when the pump does not deliver medicament to the patient the controller may check the value of ***Sa*** less frequently, i.e., once every ***t2*** seconds, where ***t2>t1.*** The value of ***t1*** may be, for example, 0.05 second, 0.1 second, or 1.0 second, etc., and the value of ***t2*** may be, for example, 5.0 seconds, 7.0 seconds, or 10.0 seconds, etc.

### Preventing mechanical damage to the RP and DP parts during partial disengagement

The invention disclosed herein may be incorporated, for example, in a wearable infusion pump device. It may occasionally occur that a user using such an infusion pump device may unintentionally, inadvertently, or accidently exert force on one of the RP and the DP, causing the two parts to disengage from one another momentarily and partially. Partly disengaging the DP from the RP means that the RP and DP are neither fully engaged nor fully disengaged. Partly separating between the DP and RP while the pump device operates (e.g., while it delivers a drug dose) may be detrimental to the operation of the pump device in terms of potential damages (e.g., wearing, breaking) to various parts of the pump device, in particular to moving parts that are involved in transferring the motor's power from the RP to the reservoir's plunger rod in the DP. To avoid this problem, the relationship between the sensor's actual output value (***Sa***) and the distance (spacing) between the RP and the DP is used to determine a 'safe distance range' ("SDR") between the RP and the DP. (An example relationship between a sensor's actual output value, ***Sa**,* and a distance, ***D,*** between a RP and a DP is shown in Fig. 18A as curve **1820.)**

Engagement of the RP and DP is regarded as 'full', hence safely operational, if the distance between the RP and the DP is within the SDR, and as 'partial', hence non-safely operational, if the distance between the RP and the DP exceeds the SDR. Accordingly, if the actual distance measured (by the pump device's controller) between the RP and the DP exceeds the SDR, the controller of the pump device may momentarily stop operation of the pump device (e.g., stop delivering a drug dose) until the distance between the RP and the DP is, again, within the SDR, which indicates full engagement between the RP and the DP being resumed.

The SDR depends on (is derived from) the actual design and configuration of the various parts involved, for example size, shape, and location of the sensor in the RP and size, shape, and location of the AMP in the DP. By way of example, the SDR may include all RP-to-DP distances between 0.6mm and 2.0mm, so that if the RP-to-DP distance momentarily exceeds 2.0mm, the pump device's (RP's) controller would pause (stop, suspend, withhold) the drug delivery, and resume delivery of the drug when the RP-to-DP distance is back within the SDR range.

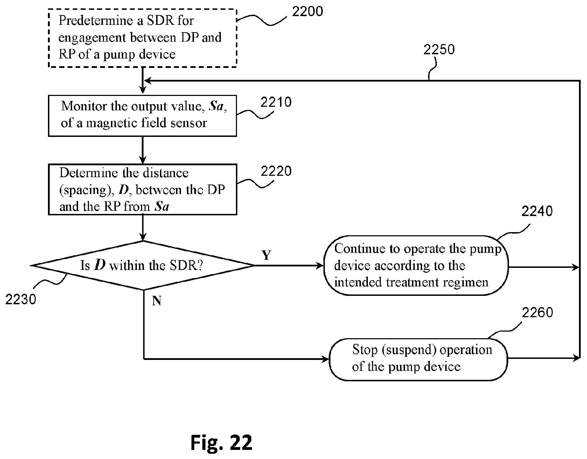

Fig. 22 shows a method of controlling an operation of a pump device according to another example embodiment. At step 2200 a safe distance range (SDR) is predetermined for engagement between a DP and a RP of a pump device.

At step 2210 the controller of the pump device monitors the output value, ***Sa**,* of the magnetic field sensor corresponding to the net magnetic field that is sensed by the magnetic field sensor, and, at step 2220, the controller determines the distance (spacing), ***D*,** between the DP and the RP from the value of ***Sa**.*

At step 2230 the controller compares the distance, ***D*,** to the predetermined SDR, and determines whether the value of ***D*** is within the predetermined SDR. The controller may transition between operating the pump device and pausing, or suspending, operation of the pump device based on the comparison result. That is, if the value of ***D*** is within the predetermined SDR (the condition is shown as "Y" at step 2230), the controller continues to operate, at step 2240, the pump device normally for example according to the intended treatment regimen and continue to monitor (2250) the value of ***Sa*** in order to detect changes, should there be any, in the distance ***D*.** If the value of ***D*** exceeds the predetermined SDR (the condition is shown as "N" at step 2230), the controller stops (suspends), at step 2260, operation of the pump device as a precaution measure to prevent damages to the moving parts of the DP or RP, or to both parts of the pump device. Operating the pump device when the distance ***D*** is within the SDR may include executing a treatment regimen. Stopping, or suspending, operation of the pump device when the distance ***D*** exceeds the SDR may include pausing execution of the treatment regimen until the distance ***D*** between the RP and DP is back within the SDR range.

Referring again to step 2230, the example transition criterion that the pump device's controller uses to determine whether operation of the pump device should proceed normally (e.g., per step 2240) or be suspended (e.g., per step 2260) is based on a comparison of the distance, ***D*,** between the DP and the RP of the pump device to the safe distance range (SDR). However, the transition criterion may also factor in the time factor. Namely, if the distance, ***D*,** between the DP and the RP of the pump device exceeds the SDR the controller may use a transition parameter that is a function of, for example, the deviation of the distance, ***D*,** between the DP and RP from the SDR, and the duration of the deviation. For example, if the value of ***D*** exceeds the SDR, the smaller the deviation of the distance ***D*** from the SDR (i.e., the closer is ***D*** to the SDR), the longer the time that the pump device can be at this state before the controller stops or suspends operation of the pump device. Inversely, if the value of ***D*** exceeds the SDR, the larger the deviation of the distance ***D*** from the SDR, the shorter the time that the controller allows the pump device to be in this state before the controller stops or suspends operation of the pump device. The rationale behind this is that the closer the distance, ***D,*** to the SDR, the lower the potential damage to the DP and to the RP, so the controller may allow the pump device to stay at this state for an extended period of time without risking the integrity of the pump device. Inversely, the greater the deviation of the distance, ***D,*** from the SDR, the greater the potential damage to the DP and to the RP, so the controller may allow the pump device to stay at the 'risky' state only for a relatively short period of time in order to avoid risking the integrity (e.g., mechanical integrity) of the pump device.

### Positioning of the magnetic field sensor: dipole magnet versus multipole magnet

As described herein, when the DP and RP of the pump device are disengaged, the net magnetic field that the magnetic field sensor (e.g., Hall effect sensor) senses should be zero irrespectively of the number of magnetic poles or magnetization direction(s) of the magnet. To facilitate this feature, the magnetic field sensor is centered in the magnet and positioned with its sensing plane arranged according to the magnetization configuration (e.g., dipole, 4-pole) of the magnet. For example, in case of dipole magnetization (Figs. 2A-2B), the magnetic field sensor **(230, 270)** is positioned with its sensing plane arranged parallel (or generally parallel) to the magnetic field flux lines **(220, 260)** that are produced by the dipole magnet **(210, 250),** such that the sensor's sensing plane is perpendicular to the Y-axis in the drawings (Figs. 2A-2B). In case of 4-pole magnetization (Figs. 4A-4B), the magnetic field sensor **(450)** is positioned with its sensing plane arranged perpendicular (or generally perpendicular) to the magnetic field flux lines **(440, 460)** that are produced by the 4-pole magnet (magnet **410),** and to the Y-axis in the drawings (Figs. 4A-4B). So, the sensor's sensing area/plane is arranged perpendicular to the Y-axis regardless of the number of magnetic poles (dipole or multipole) or the magnetization configuration of the magnet.

The difference between dipole magnetization and multipole magnetization (the 4-pole magnetization described herein is a special case of multipole magnetization) is the undisrupted (i.e., the genuine) direction(s) of the magnetic field flux lines with respect to the sensor's sensing plane. ('Undisrupted direction', or 'original direction(s)', means the direction(s) of magnetic field flux lines when the DP and the RP of the pump device are disengaged. The undisrupted direction(s) of the magnetic fields change (are disrupted) at the magnetic field sensor, i.e., deflected/redirected, by the AMP as the DP and RP of the pump device are engaged.)

### AMPs, magnets and sensors

To facilitate generation of a magnetic attraction force between an AMP and a magnet, the AMP is made from a ferrous material. For example, iron, cobalt and nickel, as well as alloys composed of these ferromagnetic metals, are strongly attracted to magnets. As described herein, in addition to inducing magnetic attraction force AMPs have another function, which is deflecting (redirecting) magnetic field lines at the magnetic field sensor when the DP and RP of the pump device are engaged. To facilitate the latter feature, the material selected for the AMP is characterized by having a relatively high magnetic permeability. (The higher the magnetic permeability of an AMP, the greater the number of magnetic force lines deflected by the AMP, hence the greater the deflection effect of the AMP.) So, AMPs include a metal that is magnetizable and conducts magnetic flux when they are near the magnet (i.e., when the DP is engaged with the RP of the pump device) but they do not become magnetized by the magnet or conduct magnetic flux when the AMP is distanced away from the magnet (i.e., when the DP is disengaged from the RP of the pump device).

The magnet shown in various drawings (for example magnet **110** in Fig. 1, magnet **250** in Fig. 2B, magnet **410** in Fig. 4A, magnet **1410** in Fig, 14, magnet **1550** in Figs. 15A-15B, and magnet **1610** in Fig. 16A) and described herein is a permanent magnet. Permanent magnets differ from temporary magnets by their ability to remain magnetized without the influence of a nearby external magnetic field. The magnet mentioned herein may be a neodymium-iron-boron (NdFeB, Nd₂Fe₁₄B) magnet, which is currently considered the strongest of the permanent magnets.

The magnetic field sensor shown in various drawings (for example sensor **120** in Fig. 1, sensor **270** in Fig. 2B, sensor **450** in Fig. 4A, sensor 570 in Fig. 5C, sensor 650 in Fig. 6B, sensor **1420** in Fig. 14, sensor **1560** in Figs. 15A-15B, and sensor **1620** in Figs. 16A-16B) and described herein may be a 1-axis magnetic sensor, a 2-axis sensor, a 3-axis sensor, a Hall effect sensor, a semiconducting magneto-resistor, a ferromagnetic magneto-resistor, a fluxgate sensor, an induction magnetometer, a permanent magnet linear contactless displacement sensor, a magneto-resistive position sensor, a magnetic force and torque sensor, and the like. The magnet-metal plate-sensor setup may be designed to accommodate for the type of magnetic sensor used.

The articles "a" and "an" are used herein to refer to one or to more than one (e.g., to at least one) of the grammatical object of the article, depending on the context. By way of example, depending on the context, "an element" can mean one element or more than one element. The term "including" is used herein to mean, and is used interchangeably with, the phrase "including but not limited to". The terms "or" and "and" are used herein to mean, and are used interchangeably with, the term "and/or," unless context clearly indicates otherwise. The term "such as" is used herein to mean, and is used interchangeably, with the phrase "such as but not limited to".

Having thus described exemplary embodiments of the invention, it will be apparent to those skilled in the art that modifications of the disclosed embodiments will be within the scope of the invention. Alternative embodiments may, accordingly, include functionally equivalent objects/articles. For example, an asymmetrical metal plate (AMP) may have a different design (e.g., different shape, size and/or material) comparing to the AMPs described herein and shown in the drawings, provided that the different designs of the AMP function in the way(s) described herein. Typically, the disposable part of the pump device may include one medicament reservoir or two medicament reservoirs, and each medicament reservoir may contain levodopa or carbidopa, or a combination of levodopa and carbidopa. Any permanent magnet may be used, provided that it functions in the way described herein. Features of certain embodiments may be used with other embodiments shown herein. The present disclosure is described in connection with pump devices that include a DP and a RP. However, the present disclosure may be relevant to (e.g., it may be implemented by, used with or for) other types of 'two-part' devices, such as pumps, syringes, therapeutic drug dispensing devices, and the like. Hence the scope of the claims that follow is not limited by the disclosure herein.

Various aspects and embodiments may be understood with reference to the following numbered clauses, in which:
1. A pump device for delivering fluid medicament(s) to a user, the pump device comprising:
   a reusable part comprising:
      a magnetic field sensor having a magnetic field sensing area;
      a magnet circumferentially surrounding the magnetic field sensor, said magnet magnetized to produce one or more magnetic fields in direction(s) that zero out a net magnetic field that is sensed by the magnetic field sensor, and
      a controller to read an output value of the magnetic field sensor; and
   a disposable part magnetically engageable with the reusable part, the disposable part comprising:
      a metal plate configured to be magnetically attractable to the magnet to magnetically engage the disposable part with the reusable part, and, during the engagement, to magnetically deflect one or more of the one or more magnetic fields at the magnetic field sensing area such that the net magnetic field sensed by the magnetic field sensor is greater than zero,
   wherein the controller is configured to determine an engagement state between the disposable part and the reusable part from an output value **(*Sa*)** of the magnetic field sensor corresponding to the sensed net magnetic field.
2. The device of clause 1, wherein each of the magnetic field sensing area, the magnet and the metal plate forms a plane that coincides with, or is parallel to, an X-Z plane of the Cartesian coordinate system and perpendicular to a Y-axis of the Cartesian coordinate system.
3. The device of clause 2, wherein the magnet comprises a central aperture and wherein the magnetic field sensor is centered in the aperture of the magnet at a point coinciding with the origin of the Cartesian coordinate system.
4. The device of clause 2, wherein the metal plate is asymmetrical with respect to an asymmetry line coinciding with the Z-axis, the asymmetry line dividing the metal plate into a primary section and an auxiliary section, wherein the primary section is configured to induce a magnetic attraction force between the primary section and the magnet when the disposable part is brought into proximity to the reusable part, and, at the same time, deflect the one or more of the one or more magnetic fields, and wherein the auxiliary section is configured to induce a magnetic attraction force between the auxiliary section and the magnet when the disposable part is brought into proximity to the reusable part.
5. The device of clause 1, wherein the metal plate unevenly deflects the one or more of the one or more magnetic fields to make the deflection detectable by the controller.
6. The device of clause 1, wherein the metal plate is configured such that the magnetic field sensor does not enter saturation when the disposable part is engaged with the reusable part, and wherein the controller is configured to distinguish the engagement state from a faulty condition associated with the magnetic field sensor entering saturation.
7. The device of clause 1, wherein the disposable part comprises one medicament reservoir, and wherein the controller is configured to compare the output value **(*Sa*)** of the magnetic field sensor to a threshold value ***(Sth)*** to determine the engagement state.
8. The device of clause 1, wherein the controller is configured to check the value of ***Sa*** once every ***t1*** seconds when the disposable part and reusable part of the pump device are engaged or the pump device actually delivers medicament to a patient, and once every ***t2*** seconds (where ***t2>t1*)** when the pump does not deliver medicament to the patient.
9. The device of clause 1, wherein the disposable part comprises two medicament reservoirs, and wherein the controller is configured either -
   to compare the output value **(*Sa*)** of the magnetic field sensor to a null value (***Sₙᵤₗₗ***) of the magnetic field sensor to distinguish between engagement of the disposable part and the reusable part in a first engagement orientation ('SIDE-B') and engagement of the disposable part and reusable part in a second engagement orientation ('SIDE-A'), or
   to compare the output value **(*Sa*)** of the magnetic field sensor to a first threshold value **(*Sth1*)** to determine engagement of the disposable part in a first engagement orientation ('SIDE-B'), and to a second threshold value **(*Sth2*)** to determine engagement in a second engagement orientation ('SIDE-A'), wherein ***Sth1**>**Sₙᵤₗₗ*** and ***Sth2**<**Sₙᵤₗₗ**.*
10. The device of clause 2, wherein the disposable part comprises two medicament reservoirs, and wherein a first engagement orientation ('SIDE-B') of the disposable part is detectably distinguishable from a second engagement orientation ('SIDE-A') of the disposable part due to the metal plate being asymmetrical with respect to an asymmetry line coinciding with the Z-axis.
11. The device of clause 9, wherein the controller is configured to output, audibly and/or visually, an indication regarding engagement between the disposable part and the reusable part and/or correctness of the engagement orientation.
12. The device of clause 1, wherein the magnet is a permanent magnet.
13. The device of clause 1, wherein the magnet is a dipole magnet magnetized diametrically.
14. The device of clause 13, wherein the dipole magnet is configured to produce a magnetic field that is parallel to the magnetic field sensing area such that the net magnetic field sensed by the magnetic field sensor is zero, or near zero.
15. The device of clause 14, wherein during engagement of the disposable part with the reusable part the net magnetic field sensed by the magnetic field sensor is greater than zero due to the metal plate deflecting the magnetic field at the magnetic field sensing area.
16. The device of clause 1, wherein the magnet is a multipole magnet comprising a number **'*n*'** (***n***=1, 2, 3,...) of pairs of conjugated magnetic poles (N/S), wherein the magnet is magnetized diametrically, or axially, or both diametrically and axially.
17. The device of clause 16, wherein the multipole magnet is configured to produce multiple magnetic fields in opposite directions at the magnetic field sensing area such that the net magnetic field sensed by the magnetic field sensor is zero, or near zero, due to mutual cancellation of opposing magnetic fields at the magnetic field sensing area.
18. The device of clause 17, wherein the multipole magnet is a 4-pole magnet comprising a first pair of conjugate magnetic poles (N/S) that is axially magnetized in a first direction, and a second pair of conjugate magnetic poles (N/S) that is axially magnetized in a second direction opposite the first direction.
19. The device of clause 18, wherein during engagement of the disposable part with the reusable part the magnetic field sensed by the magnetic field sensor is greater than zero due to the metal plate deflecting the magnetic fields at the magnetic field sensing area.
20. The device of clause 4, wherein the primary section and the auxiliary section of the asymmetrical metal plate (AMP) are separate, unconnected, sections.
21. The device of clause 4, wherein the primary section and the auxiliary section of the asymmetrical metal plate (AMP) form one monolithic object.
22. The device of clause 4, wherein the primary section of the asymmetrical metal plate (AMP) comprises a primary tab, said primary tab extending inwardly in said AMP, towards the auxiliary section of the AMP.
23. The device of clause 22, wherein the auxiliary section of the AMP comprises an auxiliary tab, the auxiliary tab extending inwardly in said AMP, towards the primary tab.
24. The device of clause 23, wherein the primary tab extends inwardly more than the auxiliary tab, and has a greater surface area than the auxiliary tab.
25. The device of clause 4, wherein the primary section of the AMP is configured to partially overlap the magnetic field sensing area when the disposable part and the reusable part are engaged, with the partial overlapping percentage being ***P***[%].
26. The device of clause 25, wherein ***P***=50% (±10%).
27. The device of clause 25, wherein the value of ***P***[%] is a tradeoff between a magnetic attraction force to be induced between the magnet and the metal plate, and a magnetic deflection to be induce by the metal plate in the magnetic field(s) at the magnetic field sensing area.
28. The device of clause 27, wherein the value of ***P***[%] is selected such that the magnetic field sensor does not enter saturation when the disposable part and the reusable part are engaged, to enable the controller to distinguish the engagement state from a faulty condition causing the magnetic field sensor to enter saturation.
29. The device of clause 4, wherein the design of the asymmetrical metal plate is a tradeoff between a magnetic attraction force to be induced between the magnet and the asymmetrical metal plate, and a magnetic field deflection to be induce by the asymmetrical metal plate in the magnetic field(s) at the magnetic field sensing area.
30. The device of clause 1, wherein the controller is further configured to: (1) determine a distance between the disposable part and the reusable part from the output value **(*Sa*)** of the magnetic field sensor corresponding to the sensed net magnetic field, (2) compare said distance to a safe distance range (SDR), and (3) transition between operating the pump device and suspending operation of the pump device based on the comparison result.
31. The device of clause 30, wherein the controller is configured to operate the pump device if said distance is within the SDR or suspend operation of the pump device if said distance exceeds the SDR.
32. The device of clause 30, wherein the controller is configured to use a transition parameter to transition between operation of the pump device and suspension of the operation of the pump device, wherein the transition parameter is a function of: (1) a value of the distance, ***D,*** between the DP and the RP, and (2) the time, ***T*,** during which the distance, ***D,*** between the DP and the RP has that value.
33. The device of clause 30, wherein operating the pump device comprises executing a treatment regimen, and wherein suspending operation of the pump device comprises suspending execution of the treatment regimen.
34. The device of any of clauses 7 and 9, wherein the medicament reservoir contains levodopa or carbidopa, or a combination of levodopa and carbidopa.

## Claims

1. A pump device for delivering fluid medicament(s) to a user, the pump device comprising:
a reusable part comprising:
a magnetic field sensor having a magnetic field sensing area;
a magnet circumferentially surrounding the magnetic field sensor, and
a controller to read an output value of the magnetic field sensor; and
a disposable part magnetically engageable with the reusable part, the disposable part comprising:
a metal plate configured to be magnetically attracted to the magnet to magnetically engage the disposable part with the reusable part, and, during the engagement, to magnetically deflect one or more magnetic fields originating from the magnet at the magnetic field sensing area,
wherein the controller is configured to determine an engagement state between the disposable part and the reusable part from an output value of the magnetic field sensor corresponding to a sensed net magnetic field resulting from said one or more magnetic fields originating from the magnet.

2. The device of claim 1, wherein the magnet comprises a central aperture and wherein the magnetic field sensor is centered in the aperture of the magnet.

3. The device of claim 1 or 2, wherein the metal plate is asymmetrical with respect to an asymmetry line, the asymmetry line dividing the asymmetrical metal plate (AMP) into a primary section and an auxiliary section, wherein the primary section is configured to induce a magnetic attraction force between the primary section and the magnet when the disposable part is brought into proximity to the reusable part, and, at the same time, deflect one or more of the one or more magnetic fields, and wherein the auxiliary section is configured to induce a magnetic attraction force between the auxiliary section and the magnet when the disposable part is brought into proximity to the reusable part.

4. The device of any preceding claim, wherein the metal plate unevenly deflects one or more of the one or more magnetic fields to make the deflection detectable by the controller.

5. The device of any preceding claim, wherein the disposable part comprises one medicament reservoir, and wherein the controller is configured to compare the output value (***Sa***) of the magnetic field sensor to a threshold value (***Sth***) to determine the engagement state.

6. The device of any preceding claim, wherein the disposable part comprises two medicament reservoirs, and wherein the controller is configured either:
to compare the output value (***Sa***) of the magnetic field sensor to a null value (***Sₙᵤₗₗ***) of the magnetic field sensor to distinguish between engagement of the disposable part and the reusable part in a first engagement orientation ('SIDE-B') and engagement of the disposable part and reusable part in a second engagement orientation ('SIDE-A'), or
to compare the output value (***Sa***) of the magnetic field sensor to a first threshold value (***Sth1***) to determine engagement of the disposable part in a first engagement orientation ('SIDE-B'), and to a second threshold value (***Sth2***) to determine engagement in a second engagement orientation ('SIDE-A'), wherein ***Sth1**>**Sₙᵤₗₗ*** and ***Sth2**<**Sₙᵤₗₗ**.*
